# EUROPEAN PATENT SPECIFICATION

(11) **EP 2 661 508 B1**
(45) Date of publication and mention of the grant of the patent: **12.09.2018**
(21) Application number: 12704144.0
(22) Date of filing: 09.01.2012
(51) Int. Cl.: C12Q 1/70

(54) **METHOD FOR GENOTYPING AND QUANTIFYING A HIGH-RISK HUMAN PAPILLOMAVIRUS**
VERFAHREN ZUR GENOTYPIERUNG UND QUANTIFIZIERUNG EINES HOCHRISKANTEN MENSCHLICHEN PAPILLOMAVIRUS
PROCÉDÉ POUR LE GÉNOTYPAGE ET LA QUANTIFICATION D'UN PAPILLOMAVIRUS HUMAIN À HAUT RISQUE

(30) Priority: 07.01.2011 US 201161430797 P
(43) Date of publication of application: 13.11.2013
(73) Proprietor: QIAGEN Gaithersburg, Inc., Gaithersburg, MD 20878 (US)
(72) Inventor: RANGWALA, Sameera, Gaithersburg MD 20878 (US); KOBAYASHI, Lori, Boonsboro MD 21713 (US); GAY, Tanya, Gaithersburg MD 20878 (US)
(74) Representative: Roth, Carla
(86) International application number: PCT/US2012/020684
(87) International publication number: WO 2012/094682

(56) References cited:
- EP-A2- 1 806 410
- WO-A1-2008/139938
- WO-A1-2009/057993
- WO-A1-2010/004251
- US-A1- 2005 032 038
- US-B2- 7 812 144
- CLAD ANDREAS ET AL: "Performance of the Aptima high-risk human papillomavirus mRNA assay in a referral population in comparison with Hybrid Capture 2 and cytology.", JOURNAL OF CLINICAL MICROBIOLOGY MAR 2011 LNKD- PUBMED:21191046, vol. 49, no. 3, 29 December 2010 (2010-12-29), pages 1071-1076, XP002675145, ISSN: 1098-660X
- LI ET AL: "Detection of human papillomavirus genotypes with liquid bead microarray in cervical lesions of northern Chinese patients", CANCER GENETICS AND CYTOGENETICS, ELSEVIER SCIENCE PUBLISHING, NEW YORK, NY, US, vol. 182, no. 1, 6 March 2008 (2008-03-06) , pages 12-17, XP022513163, ISSN: 0165-4608, DOI: 10.1016/J.CANCERGENCYTO.2007.12.007
- GHEIT TARIK ET AL: "Development of a sensitive and specific assay combining multiplex PCR and DNA microarray primer extension to detect high-risk mucosal human papillomavirus types", JOURNAL OF CLINICAL MICROBIOLOGY, AMERICAN SOCIETY FOR MICROBIOLOGY, WASHINGTON, DC, US, vol. 44, no. 6, 1 June 2006 (2006-06-01), pages 2025-2031, XP002427648, ISSN: 0095-1137, DOI: 10.1128/JCM.02305-05
- HAN J ET AL: "Simultaneous amplification and identification of 25 human papillomavirus types with Templex technology", JOURNAL OF CLINICAL MICROBIOLOGY 200611 US LNKD- DOI:10.1128/JCM.01762-06, vol. 44, no. 11, November 2006 (2006-11), pages 4157-4162, XP002675146, ISSN: 0095-1137
- DATABASE EMBL [Online] 19 July 2007 (2007-07-19), "Sequence 25 from Patent EP1806410.", XP002675256, retrieved from EBI accession no. EMBL:CS642417 Database accession no. CS642417
- DATABASE EMBL [Online] 14 December 2010 (2010-12-14), "Sequence 26 from patent US 7812144.", XP002675257, retrieved from EBI accession no. EMBL:GX640151 Database accession no. GX640151
- DATABASE Geneseq [Online] 22 January 2009 (2009-01-22), "HPV-16 E7/E6 gene target sequence, bases 752-774.", XP002675258, retrieved from EBI accession no. GSN:ATS82292 Database accession no. ATS82292
- DATABASE Geneseq [Online] 22 January 2009 (2009-01-22), "HPV-16 E7/E6 gene target sequence, bases 698-720.", XP002675259, retrieved from EBI accession no. GSN:ATS82290 Database accession no. ATS82290
- DATABASE Geneseq [Online] 1 April 2010 (2010-04-01), "HPV16 E7 gene forward RT-PCR primer SEQ ID 49.", XP002675260, retrieved from EBI accession no. GSN:AXU96631 Database accession no. AXU96631
- DATABASE Geneseq [Online] 21 April 2005 (2005-04-21), "E7 coding region (1-87) amplifying sense PCR primer, SEQ ID NO: 37.", XP002675261, retrieved from EBI accession no. GSN:ADX15568 Database accession no. ADX15568

## Description

### FIELD OF INVENTION

The present invention relates to the detection, genotyping, and quantification of a high-risk human papillomavirus in a sample.

### BACKGROUND

Persistent infection with oncogenic high-risk (HR) human papillomavirus (HPV) genotypes is associated with increased risk for the presence of developing high-grade dysplasia or cervical carcinoma, vulvar or vaginal carcinoma. There are approximately 18 HR-HPV types that infect the genital mucosa and are defined as carcinogenic. Of these HPV types 16, 18, and 45 are associated with the highest rate of cervical adenocarcinomas. HPV types 16 and 18 account for 70 % of all cervical cancers, are the most prevalent carcinogenic HPV types, and are targeted by the new HPV vaccines.

It is well known that screening programs that utilize molecular screening for the presence of HR-HPV, either independently or as an adjunct to cytological testing, improve the efficacy for standard of care processes. Moreover, tests that quantify viral load may also be important, as increased HPV load has been shown to correlate with disease progression in cervical cancer. However, the predictive value of calculating HPV DNA load may vary depending upon the type of HPV infection present, since the biological behavior of the various HR-HPV types differ. Therefore, HR-HPV load can be a type-dependent risk marker for invasive carcinoma. As such, the ability to accurately genotype an HPV infection and quantify the viral load would be a valuable tool.

WO 2009/057993 A1 relates to the detection of HPV in cervical cancers. It describes a method wherein consensus primers are used. Clad et al. (Journal of Clinical Microbiology 2011, 49:1071) discuss the performance of the Aptima high-risk HPV mRNA assay in comparison with Hybrid Capture 2 and Cytology. Li et al. (Cancer Genetics and Cytogenetics 2008, 182:12) describe the detection of HPV genotypes with a liquid bead microarray. Gheit et al. (Journal of Clinical Microbiology 2006, 44:2025) describe an assay combining multiplex PCR and DNA microarray primer extension to detect high-risk mucosal HPV types. Han et al. (Journal of Clinical Microbiology 2006, 44:4157) relates to the simultaneous amplification and identification of 25 HPV types with Templex technology. EP 1 806 410 A2 describes fluorescent multiplex HPV-PCR assays. US 7 812 144 B2 relates to methods for detecting HPV mRNA. WO 2008/139938 A1 discloses siRNA against HPV16. WO 2010/004251 A1 concerns methods and kits for diagnosing and/or monitoring the progression of a viral disease based on methylation and provides a methylome of HPV 16.

Unfortunately, the majority of HR-HPV screening methods do not genotype for individual HPV types, but rather detect an entire group of HR-HPV. Some PCR-based assays do exist for genotyping high risk HPV types, based on amplifying a portion of the L1 region various HR-HPV genomes. However, tests targeting the L1 region are prone to false negative results because part of the L1 open reading frame is lost in a low percentage of integration events into human genome. As such, use of an L1-based HR-HPV screening program may result in the delayed monitoring and treatment of potential high risk HPV infections. Accordingly, a need exists for materials and methods for accurately genotype and/or quantifying a HR-HPV infection based on a region of the HPV genome other than L1 would be a valuable tool.

### SUMMARY

The invention can be defined by the appending claims. The invention provides for a method of genotyping a high-risk HPV according to claim 1.

Compositions, nucleic acids, assays, kits, and methods described herein address the shortcomings described above by providing nucleic acids adapted to specifically detect the E6/E7 region of high risk HPV types.

In an aspect, a nucleic acid primer or probe specific for a target sequence in the E6/E7 region of a high-risk HPV genome, wherein: a. said nucleic acid primer or probe has 80% identity or greater across its entire length to both the target sequence and at least one nucleic acid sequence in E6/E7 region of a genome of a subtype thereof; and b. said nucleic acid primer or probe does not hybridize to a nucleic acid derived from a different HPV type.

The high risk HPV genome is selected from the group consisting of HPV 16, HPV 18, and HPV 45.

In one embodiment, the nucleic acid primer comprises, consists essentially of, or consists of a sequence having about 70% to 100% identity with a sequence selected from the group consisting of SEQ ID NO:1 to SEQ ID NO:12 and SEQ ID NO:16 to SEQ ID NO:21, and/or a complement thereof.

In one embodiment, the nucleic acid probe comprises, consists essentially of, or consists of a sequence having about 70% to 100% identity with a sequence selected from the group consisting of SEQ ID NO:3, SEQ ID NO:6, SEQ ID NO:9, SEQ ID NO:12, SEQ ID NO:18, and SEQ ID NO:21, and/or a complement thereof.

In one embodiment, the primer is capable of amplifying a portion of the E6/E7 region of the HPV 16 genome, but not an E6/E7 region of HPV 18 or HPV 45 genome.

In one embodiment, the nucleic acid primer comprises, consists essentially of, or consists of SEQ ID NO:1 or SEQ ID NO:2 or a complement thereof.

In one embodiment, the primer is capable of amplifying a portion of the E6/E7 region of the HPV 18 genome, but not an E6/E7 region of HPV 16 or HPV 45 genome.

In one embodiment, the nucleic acid primer comprises, consists essentially of, or consists of SEQ ID NO:4 or SEQ ID NO:5.

In one embodiment, the primer is capable of amplifying a portion of the E6/E7 region of the HPV 45 genome, but not an E6/E7 region of HPV 16 or HPV 18 genome

In one embodiment, the nucleic acid primer of comprises, consists essentially of, or consists of SEQ ID NO:7 or SEQ ID NO:8.

In one embodiment, a primer set is provided comprising at least one nucleic acid primer comprising, consisting essentially of, or consisting of a sequence having about 70% to 100% identity with a sequence selected from the group consisting of SEQ ID NO:1 to SEQ ID NO:12 and SEQ ID NO:16 to SEQ ID NO:21, and/or a complement thereof.

In one embodiment, the primer set comprises, consists essentially of, or consists of at least one primer pair selected from the group consisting of: (a) a primer pair capable of amplifying a portion of the E6/E7 region of the HPV 16 genome, but not an E6/E7 region of HPV 18 or HPV 45 genome; (b) a primer pair capable of amplifying a portion of the E6/E7 region of the HPV 18 genome, but not an E6/E7 region of HPV 16 or HPV 45 genome; and (c) a primer pair capable of amplifying a portion of the E6/E7 region of the HPV 45 genome, but not an E6/E7 region of HPV 16 or HPV 18 genome.

In one embodiment, the primer set comprises, consists essentially of, or consists of a primer pair selected from the group consisting of: (a) a first primer having about 70% to 100% identity with SEQ ID NO: 1 and a second primer having about 70% to 100% identity SEQ ID NO: 2, (b) a first primer having about 70% to 100% identity with SEQ ID NO: 4 and a second primer having about 70% to 100% identity SEQ ID NO: 5, (c) a first primer having about 70% to 100% identity with SEQ ID NO: 7 and a second primer having about 70% to 100% identity SEQ ID NO: 8, (d) a first primer having about 70% to 100% identity with SEQ ID NO: 10 and a second primer having about 70% to 100% identity SEQ ID NO: 11, (e) a first primer having about 70% to 100% identity with SEQ ID NO: 16 and a second primer having about 70% to 100% identity SEQ ID NO: 17, (f) a first primer having about 70% to 100% identity with SEQ ID NO: 19 and a second primer having about 70% to 100% identity SEQ ID NO: 20. The primer set according to claim 13 comprising a primer pair selected from the group consisting of: (a) a first primer consisting of SEQ ID NO: 1 and a second primer having about 70% to 100% identity SEQ ID NO: 2, (b) a first primer consisting of SEQ ID NO: 4 and a second primer having about 70% to 100% identity SEQ ID NO: 5, (c) a first primer consisting of SEQ ID NO: 7 and a second primer having about 70% to 100% identity SEQ ID NO: 8, (d) a first primer consisting of SEQ ID NO: 10 and a second primer having about 70% to 100% identity SEQ ID NO: 11, (e) a first primer consisting of SEQ ID NO: 16 and a second primer having about 70% to 100% identity SEQ ID NO: 17, (f) a first primer consisting of SEQ ID NO: 19 and a second primer having about 70% to 100% identity SEQ ID NO: 20.

In another aspect, a kit for the detection of HPV 16, 18, and/or 45 is disclosed, said kit comprising a nucleic acid primer or probe specific for a target sequence in the E6/E7 region of a high-risk HPV genome, wherein: a. said nucleic acid primer or probe has 80% identity or greater across its entire length to both the target sequence and at least one nucleic acid sequence in E6/E7 region of a genome of a subtype thereof; and b. said nucleic acid primer or probe does not hybridize to a nucleic acid derived from a different HPV type.

In one embodiment, the kit comprises, consists essentially of, or consists of at least one nucleic acid primer and at least one nucleic acid probe, wherein: (a) said nucleic acid primer comprises, consists essentially of, or consists of a sequence having about 70% to 100% identity with a sequence selected from the group consisting of SEQ ID NO:1, SEQ ID NO:2, SEQ ID NO:4, SEQ ID NO:5, SEQ ID NO:7, SEQ ID NO:8, SEQ ID NO:10, SEQ ID NO:11, SEQ ID NO:16, SEQ ID NO:17, SEQ ID NO:19, and SEQ ID NO:20, or a complement thereof; and (b) said nucleic acid probe comprises, consists essentially of, or consists of a sequence having about 70% to 100% identity with a sequence selected from the group consisting of SEQ ID NO:3, SEQ ID NO:6, SEQ ID NO:9, SEQ ID NO:12, SEQ ID NO:18, and SEQ ID NO:21, or a complement thereof

In one embodiment, the kit comprises, consists essentially of, or consists of an HPV 16 primer and probe set, an HPV 18 primer and probe set, and/or an HPV 45 primer and probe set.

In one embodiment, (a) the HPV 16 primer and probe set comprises: (i) a primer having 70% to 100% identity with a sequence of SEQ ID NO:1, (ii) a primer having 70% to 100% identity with a sequence of SEQ ID NO:2, and, (iii) a probe having 70% to 100% identity with a sequence of SEQ ID NO:3; (b) the HPV 18 primer and probe set comprises: (i) a primer having 70% to 100% identity with a sequence of SEQ ID NO:4, (ii) a primer having 70% to 100% identity with a sequence of SEQ ID NO:5, and, (iii) a probe having 70% to 100% identity with a sequence of SEQ ID NO:6; and (c) the HPV 45 primer and probe set comprises (i) a primer having 70% to 100% identity with a sequence of SEQ ID NO:7, (ii) a primer having 70% to 100% identity with a sequence of SEQ ID NO:8 and, (iii) a probe having 70% to 100% identity with a sequence of SEQ ID NO:9.

In one embodiment, the kit further comprises a control primer and probe set. In one embodiment, the control primer and probe set comprises (a) a primer having 70% to 100% identity with a sequence of SEQ ID NO:16, (b) a primer having 70% to 100% identity with a sequence of SEQ ID NO:17, and, (c) a probe having 70% to 100% identity with a sequence of SEQ ID NO:18.

In another aspect, a method of genotyping a high-risk HPV is disclosed, the method comprising: (a) hybridizing at least one nucleic acid primer or probe according to claim 1 to at least a portion of a target sequence in an E6/E7 region of a first high-risk HPV genome; and (b) detecting hybridization of the nucleic acid to the E6/E7 region of the first high-risk HPV genome.

In an embodiment, the nucleic acid primer or probe comprises, consists essentially of, or consists of a sequence having about 70% to 100% identity to a sequence selected from the group consisting of SEQ ID NO:1 to SEQ ID NO:12 and SEQ ID NO:16 to SEQ ID NO:21, and/or a complement thereof

In an embodiment: (a) a nucleic acid primer is hybridized to the target sequence; and (b) hybridization is detected by a method comprising performing an amplification reaction.

In an embodiment: (a) the high-risk HPV is selected from the group consisting of HPV 16, HPV 18, and HPV 45; and (b) the amplification reaction generates an amplicon corresponding to a portion of the E6/E7 region of the HPV genome.

The nucleic acid primer may have about 70% to 100% identity across its entire length to a nucleotide sequence selected from the group consisting of: SEQ ID NO: 1; SEQ ID NO: 2; SEQ ID NO: 4; SEQ ID NO: 5; SEQ ID NO: 7; and SEQ ID NO: 8 or a complement thereof.

The method as disclosed herein, wherein a nucleic acid probe is hybridized to the target sequence, which said nucleic acid probe is optionally detectably labeled.

The method in one embodiment performed in a real time multiplex PCR format.

The method may be capable of distinguishing between HPV 16, HPV 18, and HPV 45 infections, said method comprising: (a) providing a sample suspected of comprising a high-risk HPV; (b) contacting the sample with: (i) a primer set capable of amplifying a portion of a target sequence of an E6/E7 region of the HPV 16 genome, but not an E6/E7 region of an HPV 18 or HPV 45 genome; (ii) a primer set capable of amplifying a portion of a target sequence of an E6/E7 region of the HPV 18 genome, but not an E6/E7 region of an HPV 16 or HPV 45 genome; (iii) a primer set capable of amplifying a portion of a target sequence of an E6/E7 region of the HPV 45 genome, but not an E6/E7 region of an HPV 16 or HPV 18 genome; (iv) a detectably labeled nucleic acid probe capable of hybridizing to a portion of the target sequence of the E6/E7 region of the HPV 16 genome, but not an E6/E7 region of an HPV 18 or HPV 45 genome; (v) a detectably labeled nucleic acid probe capable of hybridizing to a portion of the target sequence of the E6/E7 region of the HPV 18 genome, but not an E6/E7 region of an HPV 16 or HPV 45 genome; and (vi) a detectably labeled nucleic acid probe capable of hybridizing to a portion the target sequence of the E6/E7 region of the HPV 45 genome, but not an E6/E7 region of an HPV 16 or HPV 18 genome, wherein each nucleic acid probe has a different detectable label; (c) performing a nucleic acid amplification; and (d) detecting the presence or absence of each detectable label.

In another embodiment, the method is performed on a platform that is capable of replicating a method performed by QIAGEN's Rotor-Gene PCR instrument.

In another embodiment, the method is unaffected by the presence or absence of PreservCyt® (Hologic, Bedford, MA) and SurePath™ (Becton Dickinson, Sparks MD).

In another aspect, a composition is disclosed comprising a nucleic acid primer or probe as described herein hybridized to a target sequence in the E6/E7 region of a high-risk HPV genome.

In one embodiment, the composition comprises a high-risk HPV genome selected from the group consisting of HPV 16, HPV 18, and HPV 45.

In another embodiment, the composition comprises a primer or probe comprising, consisting essentially of, or consisting of a sequence having 70% to 100% identity with a sequence selected from the group consisting of SEQ ID NO:1 to SEQ ID NO: 12 and SEQ ID NO:16 to SEQ ID NO:21, and/or a complement thereof.

### BRIEF DESCRIPTION OF THE DRAWINGS

Figure 1 illustrates assay sensitivity of an exemplary HPV primer/probe set.
Figure 2 illustrates the results of a multiplex suppression study for exemplary primer/probe sets.
Figure 3 illustrates exemplary HPV 16 detections in both singleplex and multiplex detection assays.
Figure 4 illustrates exemplary HPV 16 detections in both singleplex and multiplex detection assays.
Figure 5 illustrates exemplary HPV 16 detections in both singleplex and multiplex detection assays.
Figure 6 illustrates the results of cycling orange (ROX) in an exemplary multiplex assay and the specific detection of HPV 16 at 10³ copies/reaction and 10⁵ copies/reaction.
Figure 7 illustrates the results (CT values) of cycling orange (ROX) in an exemplary multiplex assay and the detection of HPV 16.
Figure 8 illustrates the results of cycling yellow (HEX) in an exemplary multiplex assay and the specific detection of HPV 18 at 10³ copies/reaction and 10⁵ copies/reaction.
Figure 9 illustrates the results (CT values) of cycling orange (HEX) in an exemplary multiplex assay and the detection of HPV 18.
Figure 10 illustrates the results of cycling green (FAM) in an exemplary multiplex assay and the specific detection of HPV 45 at 10³ copies/reaction and 10⁵ copies/reaction.
Figure 11 illustrates the results (CT values) of cycling orange (FAM) in an exemplary multiplex assay and the detection of HPV 45.
Figure 12 illustrates the results of cycling red (Cy5) in an exemplary multiplex assay and the specific detection of the beta-globin as a control.
Figure 13 illustrates the results (CT values) of cycling orange (Cy5) in an exemplary multiplex assay and the detection of the control, beta-globin.
Figure 14 illustrates the results of an exemplary multiplex assay and the detection of HPV 16 at 5, 10, 100, and 10³ copies/reaction.
Figure 15 illustrates the results (CT values) of cycling orange (ROX) in an exemplary multiplex assay and the detection of HPV 16 at varying copies/reaction.
Figure 16 illustrates the results of an exemplary multiplex assay and the detection of HPV 18 at 5, 10, 100, and 10³ copies/reaction.
Figure 17 illustrates the results (CT values) of cycling yellow (HEX) in an exemplary multiplex assay and the detection of HPV 18 at varying copies/reaction.
Figure 18 illustrates the results of an exemplary multiplex assay and the detection of HPV 45 at 5, 10, 100, and 10³ copies/reaction.
Figure 19 illustrates the results (CT values) of cycling green (FAM) in an exemplary multiplex assay and the detection of HPV 45 at varying copies/reaction.

### DETAILED DESCRIPTION

The present disclosure includes methods, compositions, reagents, systems, and kits for rapidly determining the presence of a nucleic acid molecule in a sample and for quantifying the target nucleic acid molecule. The methods, compositions, reagents, systems, and kits may be used for clinical diagnostic purposes, and/or for characterizing HPV viral persistence at the genotype level, the kinetics of viral load, and/or likelihood of disease recurrence.

The methods according to the present invention, and the compositions, reagents, systems, and kits disclosed herein provide for an efficient test for initial testing or follow-up testing for genotyping and/or quantifying HPV types such as 16, 18, and/or 45. For example, methods include testing un-tested samples, verifying the presence of and/or quantifying HPV 16, 18, and/or 45 in positive samples, and verifying the absence of HPV 16, 18, and/or 45 in negative samples.

Because HPV viral loads have been correlated with disease progression in, for example, cervical cancer, HPV viral load can be a type-dependent risk marker for invasive carcinoma. Thus, methods include quantifying and categorizing viral load to diagnose risk of cancer and triage, including determining the priority of methods of treatment, etc.

Methods of the present invention also include screening samples for L1 deletions/mutations since E6 and E7 are in some cases targeted regions for determining whether a specific HPV type is present or absent.

The multiplex design of the present invention saves time and sample eluate when compared with typical individual testing/genotyping of HPV types such as 16, 18, and/or 45.

### Primers and Probes

The primers and probes of the present disclosure are capable of targeting genes in the E6 and E7 regions for HPV genotypes 16, 18, and 45. The E6 and E7 regions are suitable target regions because they are not as prone to mutagenic events during integration as the L1 region is. The primers and probes are specific for HPV 16, 18, and 45 and do not exhibit any substantial cross-reactivity among themselves or among other HPV types. The primers and probes may be sensitive in detecting the presence of HPV 16, 18, or 45 with as few as 5 copies of target sequence per reaction present.

In embodiments, the primers may be from about 15 to 50 nucleotides in length, such as from about 18 to 30, about 20 to 26, or about 22 to 24 nucleotides. By way of example and not limitation, the primers may be not more than 50 nucleotides in length, not more than 30 nucleotides in length, not more than 26 nucleotides in length, or not more than 24 nucleotides in length. The probes may be from about 15 to about 60 nucleotides in length, such as from about 18 to 40, about 20 to 35, or about 22 to 30. By way of example and not limitation, the probes may be not more than 60 nucleotides in length, not more than 40 nucleotides in length, not more than 35 nucleotides in length, or not more than 30 nucleotides in length.

Exemplary primers and probes are shown in the tables below for each of HPV types 16, 18, and 45. For type 45, two primer and probe sets are shown with set No. 1 (SEQ ID NOs 7-9) being a preferred embodiment for multiplexing analysis. In the below tables, the probe sequences are conjugated with a signal on the 5' end and a quencher on the 3' end. These molecules are shown below (bold and underlined), but they are not part of the nucleic acid sequence represented by the respective SEQ ID NOs.

**Table 1-HPV 16 Primer/Probe Set:**

| | | |
|---|---|---|
| HPV 16 primer A | SEQ ID NO. 1 | 5' AGA ACC GGA CAG AGC CCA TTA CAA 3' |
| HPV 16 primer B | SEQ ID NO. 2 | 5' GCA CAC AAT TCC TAG TGT GCC CAT 3' |
| HPC 16 Probe | SEQ ID NO. 3 | 5' **ROX-**AC GCT TCG GTT GTG CGT ACA AAG CA-**IAbRQSp** 3' |

**Table 2-HPV 18 Primer/Probe Set:**

| | | |
|---|---|---|
| HPV 18 primer A | SEQ ID NO. 4 | 5' TCATCA ACA TTT ACC AGC CCG ACG 3' |
| HPV 18 primer B | SEQ ID NO. 5 | 5' GAA ACA GCTGCT GGA ATG CTC GAA 3' |
| HPC 18 Probe | SEQ ID NO. 6 | 5' **HEX**-AGC CGA ACC ACA ACG TCA CAC AAT GT-**IABkFQ** 3' |

**Table 3-HPV 45 Primer/Probe Set No. 1:**

| | | |
|---|---|---|
| HPV 45 primer A | SEQ ID NO. 7 | 5' TTG ACG ATC CAA AGC AAC GAC CCT 3' |
| HPV 45 primer B | SEQ ID NO. 8 | 5' CCT CTG TGC GTT CCA ATG TTG CTT 3' |
| HPV 45 Probe 1 | SEQ ID NO. 9 | |

**Table 4-HPV 45 Primer/Probe Set No. 2:**

| | | |
|---|---|---|
| HPV 45 primer C | SEQ ID NO. 10 | 5' TTG TTA ATA AGG TGC CTG CGG TGC 3' |
| HPV 45 primer D | SEQ ID NO. 11 | 5' TGT TTC CCT ACG TCT GCG AAG TCT 3' |
| HPC 45 Probe 2 | SEQ ID NO. 12 | |

In some embodiments a control may be used with the above primer and probes sets, for example, a beta-globin primer and probe set may be used with the multiplex system with a fourth detectable signal. Any suitable control may be used, so long as it does not interfere with the detection and quantification of the above exemplified primer and probe sets. The following primer and probe sets may be used as controls, with set No. 1 being preferred for multiplexing analysis.

**Table 5-Beta-globin Primer/Probe Set No. 1:**

| | | |
|---|---|---|
| Beta Globin primer A | SEQ ID NO. 16 | 5' GAA GAG CCA AGG ACA GGT AC 3' |
| Beta Globin primer B | SEQ ID NO. 17 | 5' CAA CTT CAT CCA CGT TCA CC 3' |
| Beta Globin Probe 1 | SEQ ID NO. 18 | 5' CCC TAG GGT TGG CCA ATC TAC TC-**IABkFQ** 3' |

**Table 6-Beta-globin Primer/Probe Set No. 2:**

| | | |
|---|---|---|
| Beta Globin primer C | SEQ ID NO. 19 | 5' ACA CAA CTG TGT TCA CTA GC 3' |
| Beta Globin primer D | SEQ ID NO. 20 | 5' CAA CTT CAT CCA CGT TCA CC 3' |
| Beta Globin Probe 2 | SEQ ID NO. 21 | |

The primers and probes may also include those primers and probes with about 70% to 100% identity and/or homology with the various primers and probes, such as 75% or more, 80% or more, 85% or more, 90% or more, 95% or more, and/or 98% or more identity and/or homology. By way of example and not limitation, the primer and/or probe may have from 70% to 100% identity and/or homology with a sequence selected from the group consisting of SEQ ID NO: 1 to SEQ ID NO: 12 and SEQ ID NO: 16 to SEQ ID NO: 21. As a further example, the probe may have 75% or more, 80% or more, 85% or more, 90% or more, 95% or more, and/or 98% or more identity and/or homology with a sequence selected from the group consisting of SEQ ID NO: 1 to SEQ ID NO: 12 and SEQ ID NO: 16 to SEQ ID NO: 21.

In an embodiment, a nucleic acid comprising a 3' terminal sequence capable of hybridizing to nucleotides 695 to 719 of SEQ ID NO: 13 or the complement thereof is used as an HPV16 E6/E7-specific primer. By way of example and not limitation, the 3' terminal sequence comprises or consists of a sequence having 70% or more, 75% or more, 80% or more, 85% or more, 90% or more, 95% or more, and/or 98% or more identity and/or homology to nucleotides 695 to 719 of SEQ ID NO: 13 or the complement thereof. As another example, the HPV16 E6/E7-specific primer consists of a sequence having 70% or more, 75% or more, 80% or more, 85% or more, 90% or more, 95% or more, and/or 98% or more identity and/or homology to nucleotides 695 to 719 of SEQ ID NO: 13 or the complement thereof.

In another embodiment, a nucleic acid comprising a 3' terminal sequence capable of hybridizing to nucleotides 811 to 834 of SEQ ID NO. 13 or a complement thereof is used an HPV16 E6/E7-specific primer. By way of example and not limitation, the 3' terminal sequence comprises or consists of a sequence having 70% or more, 75% or more, 80% or more, 85% or more, 90% or more, 95% or more, and/or 98% or more identity and/or homology to nucleotides 811 to 834 of SEQ ID NO: 13 or the complement thereof. As another example, the HPV16 E6/E7-specific primer consists of a sequence having 70% or more, 75% or more, 80% or more, 85% or more, 90% or more, 95% or more, and/or 98% or more identity and/or homology to nucleotides 811 to 834 of SEQ ID NO: 13 or the complement thereof.

In another embodiment, a probe capable of hybridizing to nucleotides 751 to 775 of SEQ ID NO. 13 or the complement thereof is used as a probe. By way of example and not limitation, the probe comprises or consists of a sequence having 70% or more, 75% or more, 80% or more, 85% or more, 90% or more, 95% or more, and/or 98% or more identity and/or homology to nucleotides 751 to 775 of SEQ ID NO. 13 or the complement thereof.

In another embodiment, an HPV 16 E6/E7-specific primer set is provided comprising: (a) a first primer comprising a 3' terminal sequence capable of hybridizing to the complement of nucleotides 695 to 719 of SEQ ID NO: 13; and (b) a second primer comprising a 3' terminal sequence capable of hybridizing to nucleotides 811 to 834 of SEQ ID NO. 13.

In another embodiment, an HPV 16 E6/E7-specific primer/probe set is provided comprising: (a) a first primer comprising a 3' terminal sequence capable of hybridizing to the complement of nucleotides 695 to 719 of SEQ ID NO: 13; (b) a second primer comprising a 3' terminal sequence capable of hybridizing to nucleotides 811 to 834 of SEQ ID NO. 13; and (c) a probe capable of hybridizing to nucleotides 751 to 775 of SEQ ID NO. 13 or the complement thereof.

In an embodiment, a nucleic acid comprising a 3' terminal sequence capable of hybridizing to nucleotides 724 to 747 of SEQ ID NO: 14 or the complement thereof is used as an HPV18 E6/E7-specific primer. By way of example and not limitation, the 3' terminal sequence comprises or consists of a sequence having 70% or more, 75% or more, 80% or more, 85% or more, 90% or more, 95% or more, and/or 98% or more identity and/or homology to nucleotides 724 to 747 of SEQ ID NO: 14 or the complement thereof. As another example, the HPV18 E6/E7-specific primer consists of a sequence having 70% or more, 75% or more, 80% or more, 85% or more, 90% or more, 95% or more, and/or 98% or more identity and/or homology to nucleotides 724 to 747 of SEQ ID NO: 14 or the complement thereof.

In another embodiment, a nucleic acid comprising a 3' terminal sequence capable of hybridizing to nucleotides 837 to 860 of SEQ ID NO. 14 or a complement thereof is used an HPV18 E6/E7-specific primer. By way of example and not limitation, the 3' terminal sequence comprises or consists of a sequence having 70% or more, 75% or more, 80% or more, 85% or more, 90% or more, 95% or more, and/or 98% or more identity and/or homology to nucleotides 837 to 860 of SEQ ID NO. 14 or the complement thereof. As another example, the HPV18 E6/E7-specific primer consists of a sequence having 70% or more, 75% or more, 80% or more, 85% or more, 90% or more, 95% or more, and/or 98% or more identity and/or homology to nucleotides 837 to 860 of SEQ ID NO. 14 or the complement thereof.

In another embodiment, a probe capable of hybridizing to nucleotides 748 to 773 of SEQ ID NO. 14 or the complement thereof is used as a probe. By way of example and not limitation, the probe comprises or consists of a sequence having 70% or more, 75% or more, 80% or more, 85% or more, 90% or more, 95% or more, and/or 98% or more identity and/or homology to nucleotides 837 to 860 of SEQ ID NO. 14 or the complement thereof.

In another embodiment, an HPV 18 E6/E7-specific primer set is provided comprising: (a) a first primer comprising 3' terminal sequence capable of hybridizing to the complement nucleotides 724 to 747 of SEQ ID NO: 14; and (b) a second primer comprising a 3' terminal sequence capable of hybridizing to nucleotides 837 to 860 of SEQ ID NO. 14.

In another embodiment, an HPV 18 E6/E7-specific primer set is provided comprising: (a) a first primer comprising 3' terminal sequence capable of hybridizing to the complement nucleotides 724 to 747 of SEQ ID NO: 14; (b) a second primer comprising a 3' terminal sequence capable of hybridizing to nucleotides 837 to 860 of SEQ ID NO. 14; and (c) a probe capable of hybridizing to nucleotides 748 to 773 of SEQ ID NO. 14 or the complement thereof.

In an embodiment, a nucleic acid comprising a 3' terminal sequence capable of hybridizing to nucleotides 112 to 135 of SEQ ID NO: 15 or the complement thereof is used as an HPV45 E6/E7-specific primer. By way of example and not limitation, the 3' terminal sequence comprises or consists of a sequence having 70% or more, 75% or more, 80% or more, 85% or more, 90% or more, 95% or more, and/or 98% or more identity and/or homology to nucleotides 112 to 135 of SEQ ID NO: 15 or the complement thereof. As another example, the HPV45 E6/E7-specific primer consists of a sequence having 70% or more, 75% or more, 80% or more, 85% or more, 90% or more, 95% or more, and/or 98% or more identity and/or homology to nucleotides 112 to 135 of SEQ ID NO: 15 or the complement thereof.

In another embodiment, a nucleic acid comprising a 3' terminal sequence capable of hybridizing to nucleotides 208 to 231 of SEQ ID NO. 15 or a complement thereof is used an HPV45 E6/E7-specific primer. By way of example and not limitation, the 3' terminal sequence comprises or consists of a sequence having 70% or more, 75% or more, 80% or more, 85% or more, 90% or more, 95% or more, and/or 98% or more identity and/or homology to nucleotides 208 to 231 of SEQ ID NO. 15 or the complement thereof. As another example, the HPV45 E6/E7-specific primer consists of a sequence having 70% or more, 75% or more, 80% or more, 85% or more, 90% or more, 95% or more, and/or 98% or more identity and/or homology to nucleotides 208 to 231 of SEQ ID NO. 15 or the complement thereof.

In another embodiment, a probe capable of hybridizing to nucleotides 136 to 165 of SEQ ID NO. 15 or the complement thereof is used as a probe. By way of example and not limitation, the probe comprises or consists of a sequence having 70% or more, 75% or more, 80% or more, 85% or more, 90% or more, 95% or more, and/or 98% or more identity and/or homology to nucleotides 136 to 165 of SEQ ID NO. 15 or the complement thereof.

In another embodiment, an HPV 45 E6/E7-specific primer set is provided comprising: (a) a first primer comprising 3' terminal sequence capable of hybridizing to the complement nucleotides 112 to 135 of SEQ ID NO: 15; and (b) a second primer comprising a 3' terminal sequence capable of hybridizing to nucleotides 208 to 231 of SEQ ID NO. 15.

In another embodiment, an HPV 45 E6/E7-specific primer set is provided comprising: (a) a first primer comprising 3' terminal sequence capable of hybridizing to the complement nucleotides 112 to 135 of SEQ ID NO: 15; (b) a second primer comprising a 3' terminal sequence capable of hybridizing to nucleotides 208 to 231 of SEQ ID NO. 15; and (c) a probe capable of hybridizing to nucleotides 136 to 165 of SEQ ID NO. 15 or the complement thereof.

In an embodiment, a nucleic acid comprising a 3' terminal sequence capable of hybridizing to nucleotides 402 to 425 of SEQ ID NO: 15 or the complement thereof is used as an HPV45 E6/E7-specific primer. By way of example and not limitation, the 3' terminal sequence comprises or consists of a sequence having 70% or more, 75% or more, 80% or more, 85% or more, 90% or more, 95% or more, and/or 98% or more identity and/or homology to nucleotides 402 to 425 of SEQ ID NO: 15 or the complement thereof. As another example, the HPV45 E6/E7-specific primer consists of a sequence having 70% or more, 75% or more, 80% or more, 85% or more, 90% or more, 95% or more, and/or 98% or more identity and/or homology to nucleotides 402 to 425 of SEQ ID NO: 15 or the complement thereof.

In another embodiment, a nucleic acid comprising a 3' terminal sequence capable of hybridizing to nucleotides 546 to 569 of SEQ ID NO. 15 or a complement thereof is used an HPV45 E6/E7-specific primer. By way of example and not limitation, the 3' terminal sequence comprises or consists of a sequence having 70% or more, 75% or more, 80% or more, 85% or more, 90% or more, 95% or more, and/or 98% or more identity and/or homology to nucleotides 546 to 569 of SEQ ID NO. 15 or the complement thereof. As another example, the HPV45 E6/E7-specific primer consists of a sequence having 70% or more, 75% or more, 80% or more, 85% or more, 90% or more, 95% or more, and/or 98% or more identity and/or homology to nucleotides 546 to 569 of SEQ ID NO. 15 or the complement thereof.

In another embodiment, a probe capable of hybridizing to nucleotides 517 to 542 of SEQ ID NO. 15 or the complement thereof is used as a probe. By way of example and not limitation, the probe comprises or consists of a sequence having 70% or more, 75% or more, 80% or more, 85% or more, 90% or more, 95% or more, and/or 98% or more identity and/or homology to nucleotides 517 to 542 of SEQ ID NO. 15 or the complement thereof.

In another embodiment, an HPV 45 E6/E7-specific primer set is provided comprising: (a) a first primer comprising 3' terminal sequence capable of hybridizing to the complement nucleotides 402 to 425 of SEQ ID NO: 15; and (b) a second primer comprising a 3' terminal sequence capable of hybridizing to nucleotides 546 to 569 of SEQ ID NO. 15.

In another embodiment, an HPV 45 E6/E7-specific primer set is provided comprising: (a) a first primer comprising 3' terminal sequence capable of hybridizing to the complement nucleotides 402 to 425 of SEQ ID NO: 15; (b) a second primer comprising a 3' terminal sequence capable of hybridizing to nucleotides 546 to 569 of SEQ ID NO. 15; and (c) a probe capable of hybridizing to nucleotides 517 to 542 of SEQ ID NO. 15 or the complement thereof.

As used herein, the phrases "HPV 16 E6/E7-specific primer," "HPV 18 E6/E7-specific primer," and "HPV 45 E6/E7-specific primer" refer to nucleic acid primers capable of mediating the amplification of a portion of the E6/E7 region of the genome of the indicated HPV type, but which is not capable of mediating the amplification of a portion of the E6/E7 region of the genome of a different HPV type.

In one embodiment, the probes are designed to include a detectable signal, such as a radioactive, biotin, or fluorescent signal, suitable for the independent detection of the genotypes to be tested. Any suitable detectable signal may be used, for example, HPV 16 may be labeled with ROX, an orange fluorescent molecule; HPV 18 may be labeled with HEX, a yellow fluorescent molecule, HPV 45 may be labeled with FAM, a green fluorescent molecule, and the control, beta-globin, may be labeled with Cy5, a red fluorescent molecule. In embodiments, as shown in the tables above, the probes may be TaqMan probes with a fluorophore covalently attached to the 5' end of the probe and a quencher attached at the 3' end of the probe. When the fluorophore and quencher are in proximity, any fluorescence is inhibited by the quencher. However, when the Taq polymerase extends the primer, the probe is degraded by the exonuclease activity of the polymerase, thereby freeing the fluorophore from the quenching activity of the quencher. Any suitable combination of label and quencher may be used.

In another embodiment, the probe is utilized to generate a DNA:RNA hybrid for use in hybrid capture. Hybrid capture technology utilizes certain antibodies capable of binding to DNA:RNA hybrids in various methods of purifying and detecting specific target nucleic acids in a sample. Various iterations of the hybrid capture method are described in, *inter alia*, U.S. Patent Nos. 5,994,079, 6,027,897, 6,277,579, 6,686,151, and 7,439,016; US Patent Publication Nos. 2006/0051809 A1, 2009/0162851 A1, and 2009-0298187 A1; and PCT Publication No. WO 01/96608. The basic hybrid capture protocol comprises: (1) hybridizing a nucleic acid probe to the target nucleic acid to generate a DNA:RNA hybrid; (2) associating the DNA:RNA hybrid with a solid phase to facilitate isolation of the target nucleic acid; and (3) detecting the DNA:RNA hybrid. In various iterations, anti-DNA:RNA hybrid antibodies can be used in either step (2) or step (3). By way of example and not limitation, the anti-DNA:RNA hybrid antibody may be bound to the solid phase (covalently or otherwise), thereby mediating "capture" of the DNA:RNA hybrid to the solid phase. Alternatively, a nucleic acid probe bound to the solid phase (covalently or otherwise) may capture the DNA:RNA hybrid to the solid phase, which may then be detected by a detectably labeled anti-DNA:RNA hybrid antibody.

### Specimen Preparation

Any suitable specimen preparation method may be used. When the methods are used to verify or quantify a positive or negative result, a sample of the already purified specimen may be used. When the methods are used to make an initial determination/quantification of HPV 16, 18, and/or 45, specimens may be purified using any suitable sample purification method.

### PCR Equipment and Conditions

Any suitable PCR equipment and conditions capable of real-time PCR analysis may be used to amplify and detect the target PCR product. In embodiments, a Rotor-Gene Q, made by Qiagen, may be used.

### EXAMPLES

An HPV 16 Primer/Probe Set (SEQ ID NOs. 1-3), HPV 18 Primer/Probe Set (SEQ ID NOs. 4-6), and HPV 45 Primer Probe Set No. 1 (SEQ ID NOs. 7-9) were evaluated in singleplex real time PCR. Analytical specificity against both high risk (HR) and low risk (LR) HPV types was tested using plasmids in a clean system at 1E+7 copies/assay. HPV types 6, 11, 16, 18, 26, 31, 33, 35, 39, 40, 42, 43, 45, 51, 52, 53, 56, 58, 59, 61, 66, 67, 68, 69, 71, 72, 73, 81, 82, 2, 3, 13, 30, 34, 44, 70, and 83 were tested. The results showed that the Primer/Probe sets were specific, respectively, for HPV 16, 18, and 45.

The Primer/Probe sets were also tested against possible interfering substances, namely, blood, contraceptive jelly, spermicide, moisturizer, hemorrhoidal anesthetic, body oil, anti-fungal cream, vaginal lubricants, and feminine spray. The results showed that these substances did not interfere with the specificity of the Primer/Probe sets.

The Primer/Probe sets were also tested against 16 non-HPV pathogens, per FDA recommendations.

The HPV 16 Primer/Probe Set, HPV 18 Primer/Probe Set, and HPV 45 Primer Probe Set No. 1 were verified by BLAST analysis. The sequences were compared to all relevant mucosal HPV types and all known subtypes of HPV 16, 18, and 45 in the NCBI database. No known subtypes of HPV 16, 18, and 45 produced greater than 10% mismatches. All known subtypes had a greater than 90% homology. Sequences with greater than 80% homology to the HPV 16, 18, and 45 primers and probes are shown in Table 7.

**Table 7- Sequences With Greater Than 80% Homology to HPV 16, 18, and 45 Primers and Probes**

| ***Sequence*** | ***HPV Type*** | ***BLAST (HPV Type)*** | ***% Identity*** |
|---|---|---|---|
| Forward Primer | *16* | 31 | 86.36 |
| Forward Primer | *18* | 97 | 87.5 |
| Forward Primer | *18* | 45 | 83.33 |
| Forward Primer | *45* | 59 | 81.82 |
| Forward Primer | *45* | 37 | 80.95 |
| Reverse Primer | *16* | 73 | 91.67 |
| Reverse Primer | *16* | 35 | 87.5 |
| Reverse Primer | *16* | 67 | 86.96 |
| Reverse Primer | *16* | 43 | 86.96 |
| Reverse Primer | *16* | 32 | 86.36 |
| Reverse Primer | *16* | 34 | 83.33 |
| Reverse Primer | *16* | 7 | 83.33 |
| Reverse Primer | *16* | 11 | 86.36 |
| Reverse Primer | *16* | 42 | 86.36 |
| Reverse Primer | *16* | 71 | 81.82 |
| Reverse Primer | *16* | 91 | 81.82 |
| Reverse Primer | *18* | 59 | 90.48 |
| Reverse Primer | *18* | 45 | 82.61 |
| Reverse Primer | *18* | 67 | 80.95 |
| Reverse Primer | *45* | 97 | 91.67 |
| Reverse Primer | *45* | 110 | 85.71 |
| Probe | *18* | 45 | 91.67 |
| Probe | *18* | 97 | 91.67 |
| Probe | *18* | 68 | 90.48 |
| Probe | *18* | 80 | 86.36 |
| Probe | *18* | 113 | 86.36 |
| Probe | *45* | 18 | 86.67 |
| Probe | *45* | 97 | 86.67 |
| Probe | *45* | 16 | 91.3 |

### Sample Preparation

Samples were purified using a modified QIAamp Media MDx Kit, which is a commercially available nucleic acid extraction and purification technology based on silica gel absorption in a column format. The modified steps from the standard protocol depending on the specimen type is shown below in Table 8.

**Table 8-Modified Protocol Depending on Specimen Type**

| **Specimen Type** | **Input Sample Volume** | **Modified Steps from Standard Protocol** | **Elution Volume** |
|---|---|---|---|
| Sample Transport Media (STM) | 100µL or 150µL denatured | Denatured specimen→ Add 50µL MES neutralization buffer | 100µL |
| PreservCyt® | 250µL+ | Centrifuge→decant→add 200µL STM | 100µL |
| SurePath™ | Pre-gradient or diluted sample: 500µL; post-gradient (from total sample volume @ 3mL): 300µL | Centrifuge→decant→add 200µL 100mM Tris-buffer pH 8 (or 9)→proteinase K incubation was extended to one hour at 56°C with shaking at 600 RPM | 100µL |

### Real-Time PCR Method

The purified samples were then used with the HPV 16 Primer/Probe Set, HPV 18 Primer/Probe Set, and HPV 45 Primer Probe Set No. 1 along with the beta-globin probe/primer Set No. 1. The mixture and conditions of the PCR method are shown below in Table 9.

**Table 9- PCR Mixture and Cycling Conditions**

| PCR Mix | |
|---|---|
| Total Reaction Volume | 25 uL |
| Template | 5 uL |
| HPV Primers | 200 nM |
| HPV Probes | 200 nM |
| Beta-globin IC Primers | 60 nM |
| Beta-globin IC Probes | 60 nM |
| QIAGEN QT Virus Master Mix (5X) | 1 X |

| Rotor-Gene Q Conditions | |
|---|---|
| Denaturation | 95° C for 5 minutes |
| Annealing | 95° C for 15 seconds |
| Extension | 60° C for 75 seconds |
| Cycles | 45 |

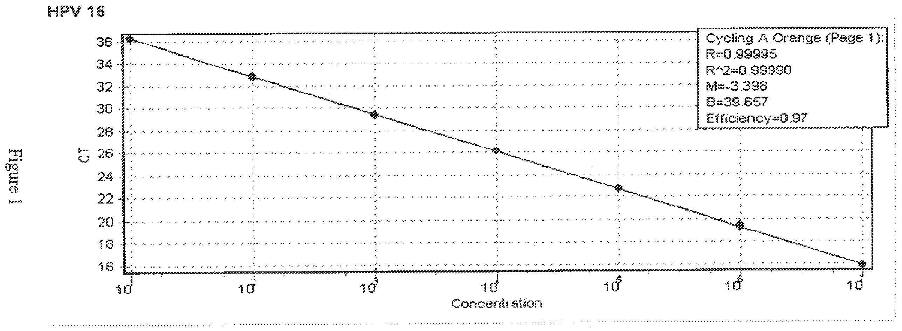

Assay Sensitivity: The dynamic range of each HPV 16, 18, and 45 multiplex primer/probe set was determined using a plasmid model. The dynamic range was determined to be at least 6 magnitudes. As illustrated in Figure 1, a correlation coefficient (R2 value) of ≥ 0.97 and PCR efficiency of ≥ .97 was consistently achieved for HPV 16, 18, and 45 from 10⁷ to 10 -genome copies/assay. Ct values and LOD for each HPV primer/probe were found to be comparable between singleplex assay detection and multiplex assay detection.

Assay Specificity: Each primer/probe pair was tested in multiplex for specificity against 27 HR and LR HPV types at 1 x 10⁷ copies/assay of each HPV type plasmid. The results are shown below in Tables 10 and 11.

**Table 10: Assay Specificity for HR HPV Types**

| | **HPV Probe Type** | | |
|---|---|---|---|
| **HR HPV Target** | **16** | **18** | **45** |
| 16 | **pos** | neg | neg |
| 18 | neg | **pos** | neg |
| 26 | neg | neg | neg |
| 31 | neg | neg | neg |
| 33 | neg | neg | neg |
| 35 | neg | neg | neg |
| 39 | neg | neg | neg |
| 45 | neg | ***neg** | pos |
| 51 | neg | neg | neg |
| 52 | neg | neg | neg |
| 56 | neg | neg | neg |
| 58 | neg | neg | neg |
| 59 | neg | neg | neg |
| 66 | neg | neg | neg |
| 68 | neg | neg | neg |
| 71 | neg | neg | neg |
| 72 | neg | neg | neg |
| 73 | neg | neg | neg |
| 82 | neg | neg | neg |

| | | | |
|---|---|---|---|
| *HPV 18 primers and probes inconsistently detected HPV 45 target input of 10⁷ with Ct value of ≥ 40 and detection of ≥ 5 copies/assay. | | | |

**Table 11: Assay Specificity for LR HPV Types**

| | **HPV Probe Type** | | |
|---|---|---|---|
| **LR HPV Target** | **16** | **18** | **45** |
| 6 | neg | neg | neg |
| 11 | neg | neg | neg |
| 40 | neg | neg | neg |
| 42 | neg | neg | neg |
| 43 | neg | neg | neg |
| 2 | neg | neg | pos |
| 3 | neg | neg | neg |
| 13 | neg | neg | neg |
| 44 | neg | neg | neg |
| 53 | neg | neg | neg |
| 67 | neg | neg | neg |
| 69 | neg | neg | neg |
| 70 | neg | neg | neg |
| 83 | neg | neg | neg |
| 30 | neg | neg | neg |
| 34 | neg | neg | neg |
| 61 | neg | neg | neg |
| 81 | neg | neg | neg |

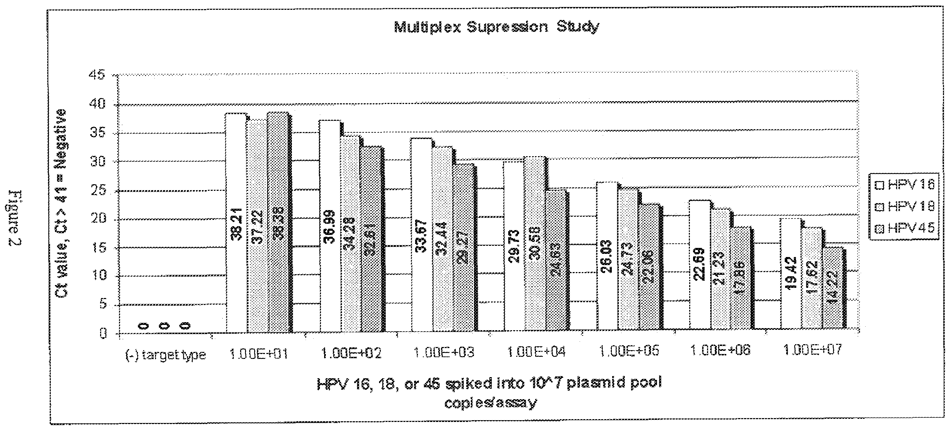

Assay Specificity-Suppression/Inhibition in Multiplex: To further test the specificity of the multiplex assay, HPV 16, 18, and 45 were diluted 10 fold from 10 copies to 10⁷ copies. These dilutions were tested in a plasmid pool of either HPV 16, 18, or 45 + HPV 39, 43, 67, and 83 at 10⁷ copies/assay of each HPV type. As illustrated in Figure 2, suppression of the assays specificity to each of the target HPV types was minimal.

Clinical Sample Performance: Over 1000 cervical specimens HC2 screened and GP PCR/LMX genotyped from STM, PC, and SP media were tested in the multiplex assay format. The GP PCR Results are shown below in Tables 12-14.

**Table 12- STM Specimen Results for GP PCR**

| **Specimen** | **HR** (+) | **LR** (+) |
|---|---|---|
| STM8517 | 16 | 40 |
| STM8545 | 16,52 | neg |
| STM8585 | 16, 18 | neg |
| STM8697 | 16,68 | neg |
| STM8643 | 16,31 | 13. 32, 69, 86, 89, 90, 6 |
| STM16 | 16 | 10, 40 |
| STM112 | 16,45 | 40, 86, 87 |
| STM8215 | 16 | neg |
| STM8790 | 16 | 81 |
| STM8705 | 16 | neg |
| STM8525 | 45 | 40 |
| STM8550 | 31 | 90 |

**Table 13- PC Specimen Results for GP PCR**

| **Specimen** | **HR (+)** | **LR** (+) |
|---|---|---|
| **PC5635** | 16, 31 | 74, 90 |
| **PC56181** | 16 | 70 |
| **PC56360** | 16, 39 | 30, 72 |
| **PC56603** | 16 | neg |
| **PC56209** | 16, 39 | neg |
| **PC56567** | 18, 16 | 40, 62, 74, 81 |
| **PC56434** | 16 | 10, 13, 32, 62, 90 |
| **PC56533** | 16, 68 | neg |
| **PC53802** | 16 | 40 |
| **PC54075** | 16, 18 | 67, 89 |
| **PC54076** | 16 | 89, 6 |

**Table 14- SP Specimen Results for GP PCR**

| | | |
|---|---|---|
| **SP10462** | 16 | neg |
| **SP10556** | 16, 52 | neg |
| **SP10583** | 16, 82 | neg |
| **SP10596** | 16 | neg |
| **SP10505** | 16, 66 | neg |
| **SP10605** | 16 | neg |
| **SP10472** | 16 | neg |
| **SP10481** | 16, 18 | neg |
| **SP10606** | 16 | 6 |
| **SP10693** | 16 | 42 |
| **SP10704** | 16, 51, 56 | neg |

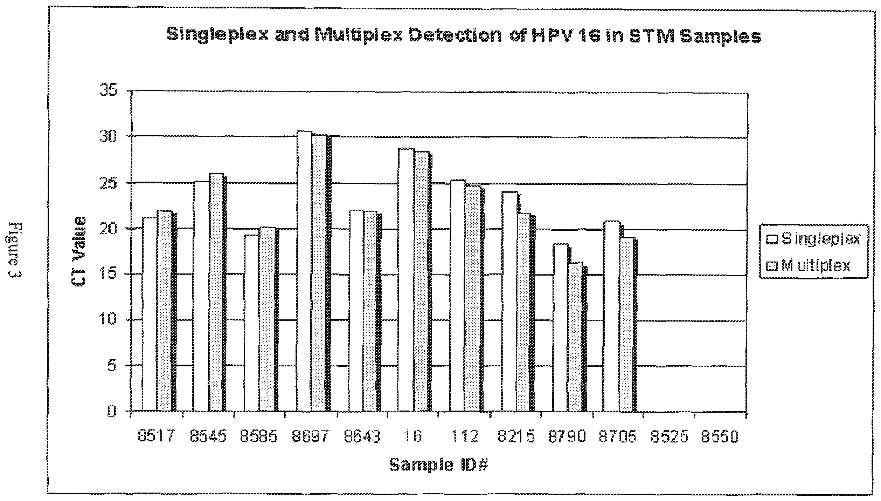

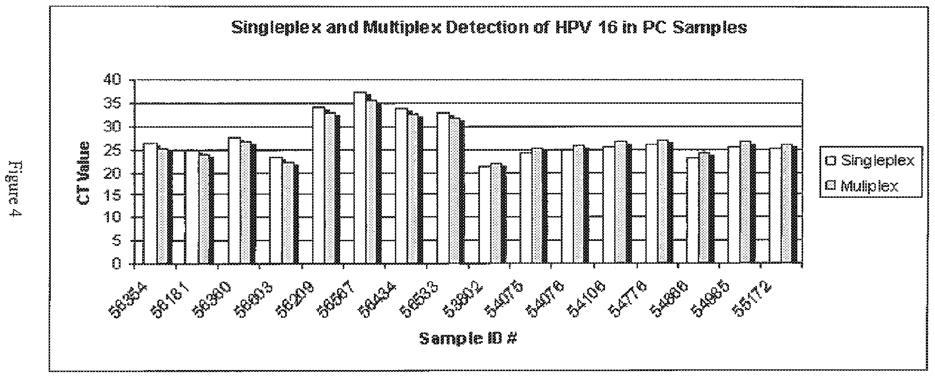

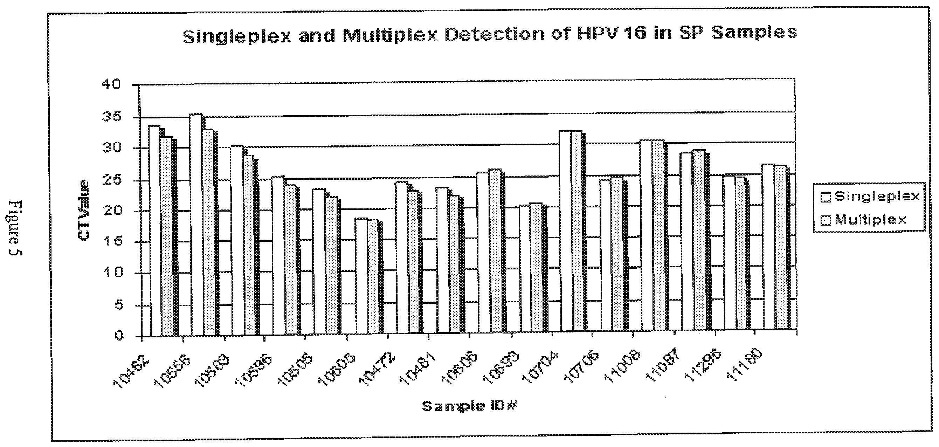

Figures 3-5 illustrate several examples of HPV 16 detection in single and multiplex assays for various sample types.

About 500 samples have been tested in both multiplex and singleplex. These tests showed comparable results in genotyping specimens positive or negative for the target HPV types (16, 18, and 45).

Clinical samples with multiple HPV infections were genotyped using GP 5+/6+ LMX Genotyping, as described in, for example, U.S. Patent No. 6,352,825. Using about 25µL of sample, the primers and probe sets for HPV 16, 18, and 45 were used to detect the presence or absence of HPV 16, 18, and 45. The results are summarized in Table 15 below.

**Table 15-Examples of Clinical Samples with Multiple Infections**

| | | **GP 5+/6+ LMX Genotyping** | | | | **qPCR, copies/∼25 uL Reaction** | | |
|---|---|---|---|---|---|---|---|---|
| **Plate Source** | **Media Type** | **Sample #** | **HR 1** | **HR 2** | **HR 3** | **HPV 16** | **HPV 18** | **HPV 45** |
| 112409AM careHPV Plate1 | DCM | 5567 | 16 | 18 | 39 | 1.21E+05 | 1.70E+05 | |
| 101909AM DCM Samples | DCM | 229 | 16 | 45 | | 1.01E+05 | | 6.31E+04 |
| 101909AM DCM Samples | DCM | 239 | 16 | 18 | 56 | 3.89E+04 | 6.20E+03 | |
| 101909AM DCM Samples | DCM | 313 | 16 | 45 | | 3.70E+03 | | 1.00E+02 |
| 101909AM DCM Samples | DCM | 373 | 18 | 39 | 45 | | 1.50E+02 | 2.50E+01 |
| 91109AM Plate 2 | DCM | 338 | 16 | 18 | 51 | 2.11E+04 | 2.66E+04 | |
| 010710AM ShubingPC | PC | 11253 | 16 | 18 | | 2.45E+02 | 2.06E+03 | |
| 113009AM Plate 1 | PC | 25136 | 16 | 18 | | 1.50E+01 | 3.50E+01 | |
| 110409AM Plate 1 Surepath | SP | 6502 | 18 | 35 | 45 | | 1.80E+03 | 2.99E+05 |
| 011210AM PL1 Haiti STM | STM | 5567 | 16 | 18 | 39 | 1.08E+04 | 6.28E+03 | |
| 011410AM PL2 Haiti STM | STM | 5941 | 16 | 18 | | 3.10E+03 | 4.20E+01 | |
| 011410AM PL2 Haiti STM | STM | 6615 | 16 | 18 | 52 | 7.50E+01 | 2.70E+01 | |
| 011210AM PL1 Haiti STM | STM | 6710 | 16 | 45 | | 2.40E+04 | | 1.26E+04 |
| 042610AM Haiti STM | STM | 7336 | 16 | 45 | | 341E+02 | | 4.23E+03 |
| Morelia | PC | 1-0138 | 16 | 45 | | 1.22E+04 | | 2.25E+03 |

4-Plex Assays: Primers and Probes for Beta-Globin were used as an internal control along with the primer/probe sets for HPV 16, 18, and 45. Various samples were run with the 4-plex primers and probes. The samples are summarized below in Table 16.

**Table 16- Samples in Run With 4-Plex To Test Specificity**

| **SAMPLE** |
|---|
| No template control (NTC) = Water |
| HPV 16 plasmid at 10³ c/rxn and 10⁵ c/rxn |
| HPV 18 plasmid at 10³ c/rxn and 10⁵ c/rxn |
| HPV 45 plasmid at 10³ c/rxn and 10⁵ c/rxn |
| Human Genomic DNA at 52 ng/rxn and 525 ng/rxn |
| Negative Clinical Pool |
| Negative Clinical Pool + HPV 16 plasmid spike of 10³ copies/rxn |
| Negative Clinical Pool + HPV 18 plasmid spike of 10³ copies/rxn |
| Negative Clinical Pool + HPV 45 plasmid spike of 10³ copies/rxn |

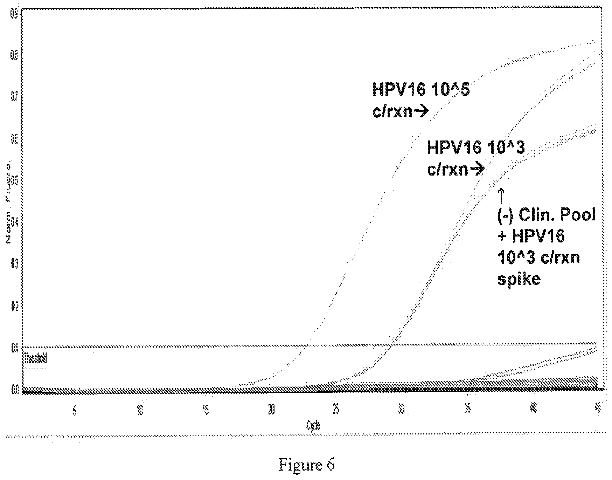

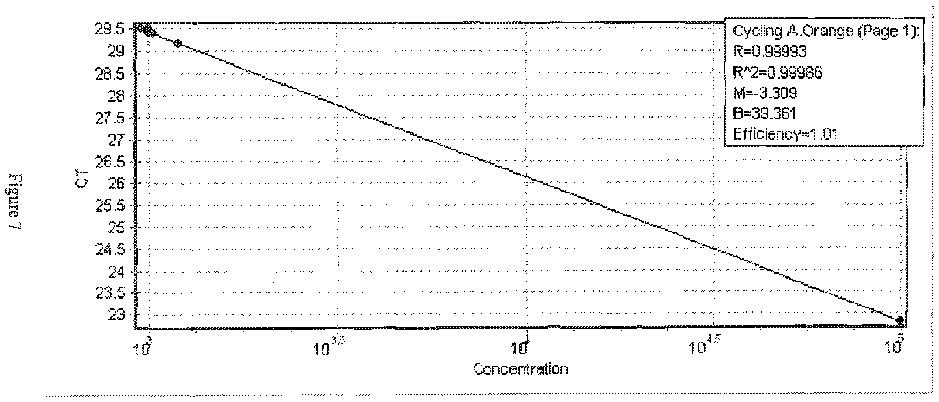

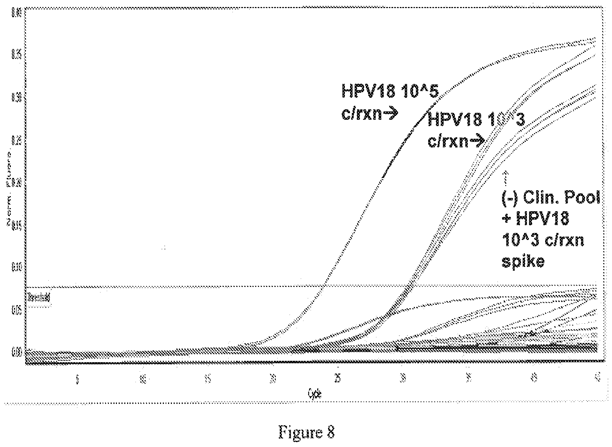

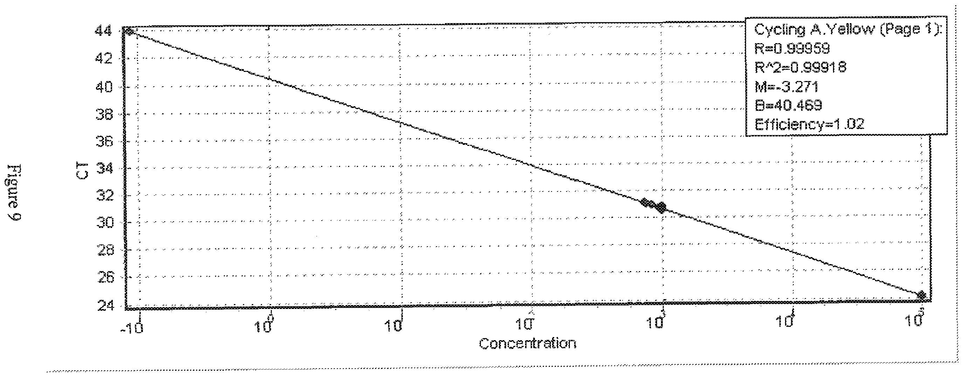

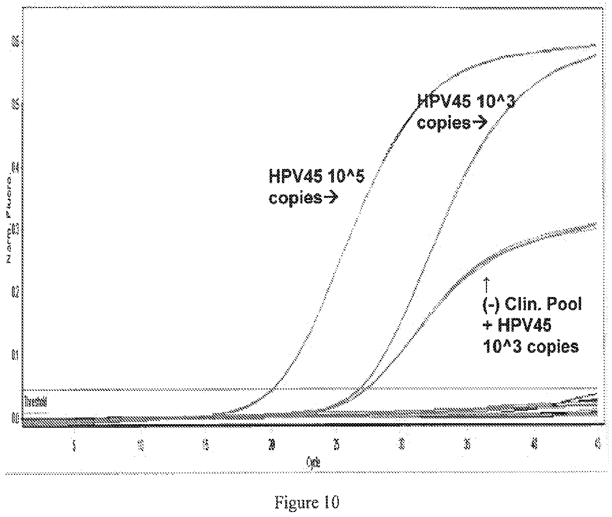

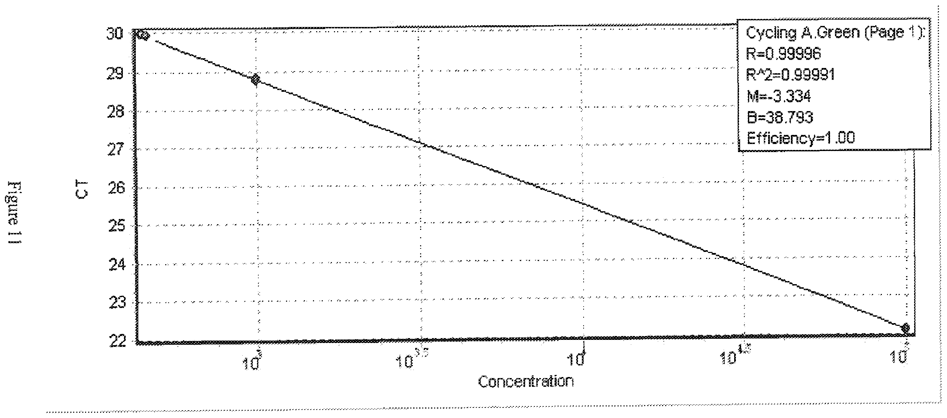

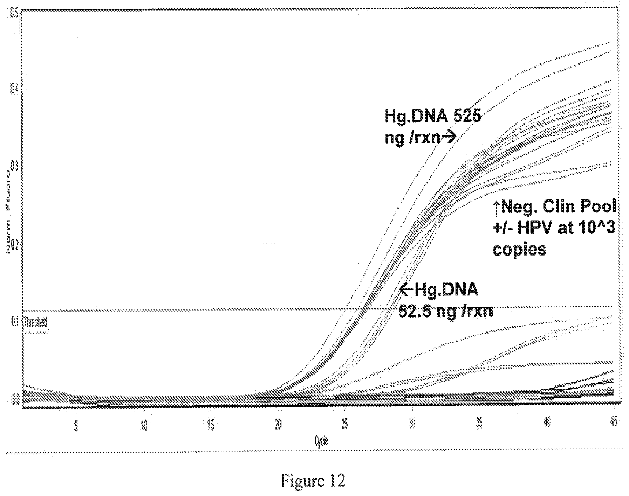

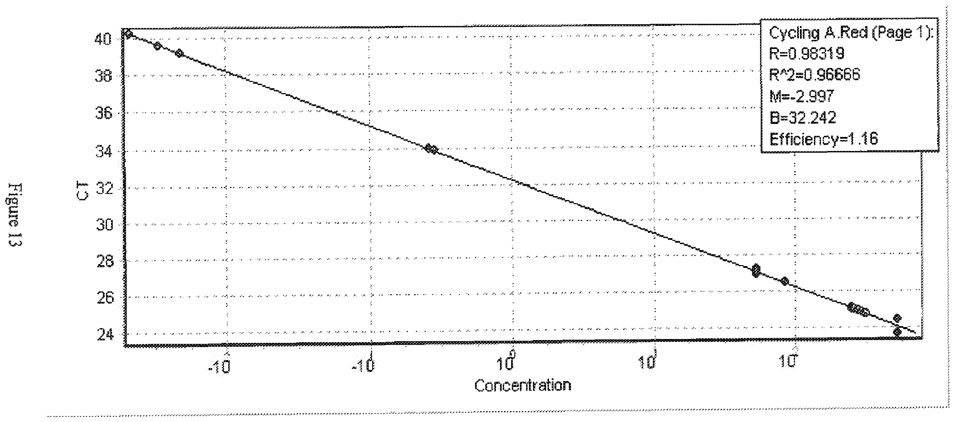

The results of running these samples are illustrated in Figures 6-13. The assays demonstrated that:
- the orange channel is specific only to samples containing HPV 16 DNA detected by the ROX probe;
- the yellow channel is specific only to samples containing HPV 18 DNA detected by the HEX probe;
- the green channel is specific only to samples containing HPV 45 DNA detected by the FAM probe;
- the red channel is specific only to negative clinical background +/- HPV 16, 18, or 45 plasmid detected by the Cy5 probe.
Thus, the specificity of each channel to the selected fluorophore in the multiplex format including the internal control primers and probes were demonstrated.

In addition to specificity, the 4-Plex assay demonstrates sensitivity in detecting the presence of HPV 16, 18, and/or 45. The following samples listed in Table 17 were run with the 4-plex primers and probes.

**Table 17-Samples in Run With 4-Plex To Test Sensitivity**

| **SAMPLES** |
|---|
| NTC = Water |
| HPV 16 plasmid at 5, 10, 100, 10³ c/rxn |
| HPV 18 plasmid at 5, 10, 100, 10³ c/rxn |
| HPV 45 plasmid at 5, 10, 100, 10³ c/rxn |

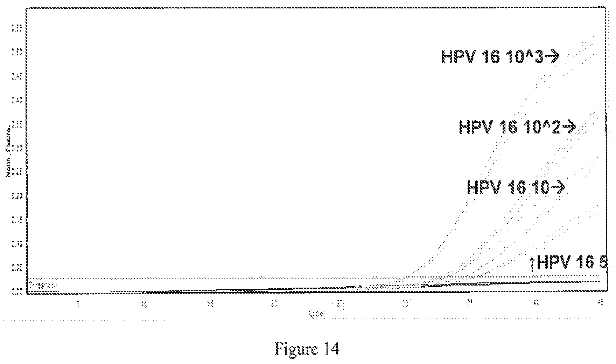

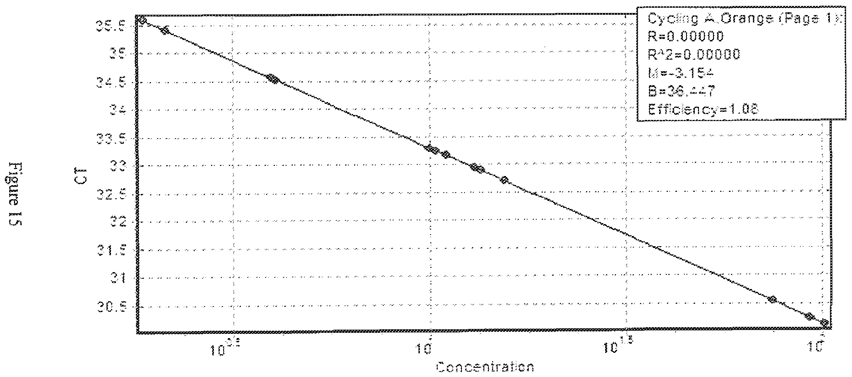

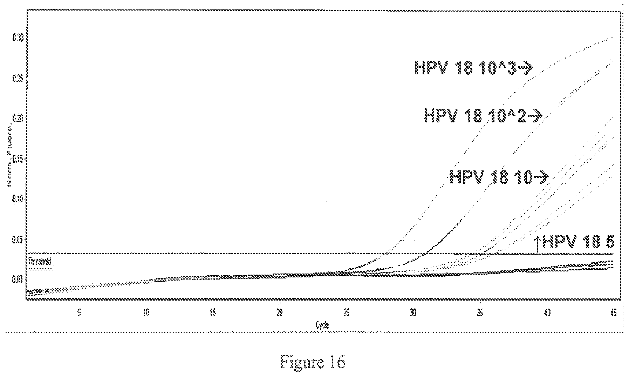

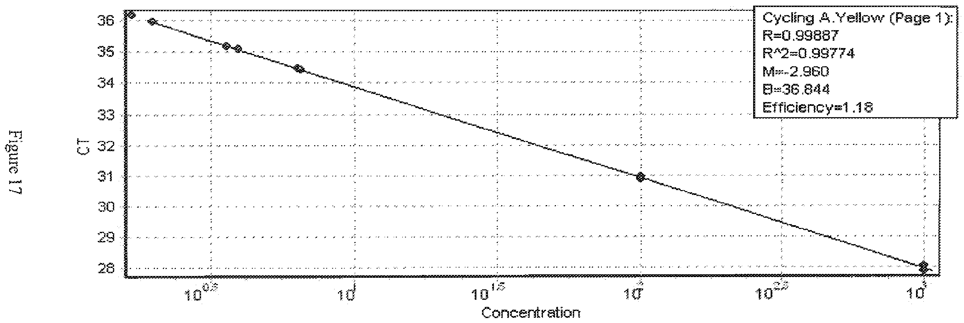

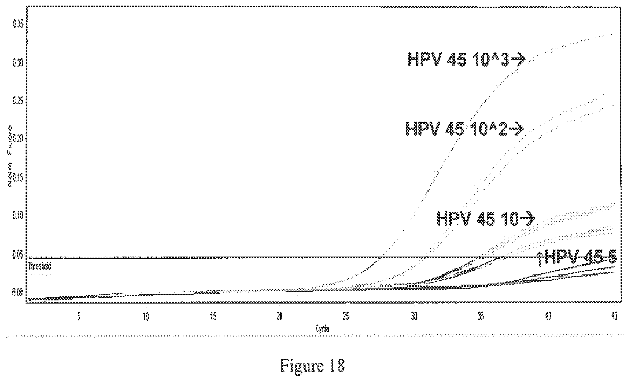

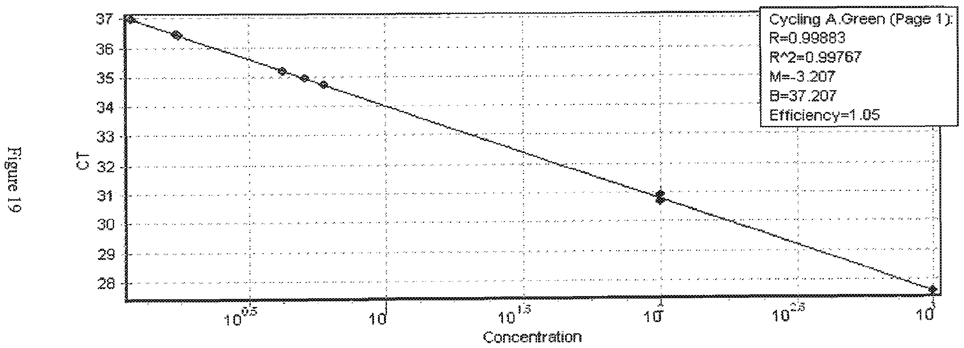

Each of the sample dilutions were spiked into a negative clinical eluate pool. The results of running these samples are illustrated in Figures 14-19. Each of the primer/probe sets demonstrated sensitivity, being detectable at levels of at least 5 copies /reaction in the presence of HPV negative clinical background.

### Sequencing Samples:

Of the numerous samples tested above, 34 samples (17 multiplex positive and 17 multiplex negative) were sequenced. All the sample sequencing matched with the multiplex data, thus providing further verification of the specificity, sensitivity, and accuracy of the multiplex assay.

## Claims

1. A method of genotyping a high-risk HPV, the method comprising:
a. hybridizing at least one nucleic acid primer to at least a portion of a target sequence in an E6/E7 region of a first high-risk HPV genome, wherein the nucleic acid primer is specific for a target sequence in the E6/E7 region of a high-risk HPV genome and wherein said nucleic acid primer has aa) 70% identity or greater across its entire length to both the target sequence and at least one nucleic acid sequence in E6/E7 region of a genome of a subtype thereof and bb) said nucleic acid primer does not hybridize to a nucleic acid derived from a different HPV type; and
b. detecting hybridization of the nucleic acid primer to the E6/E7 region of the first high-risk HPV genome, wherein hybridization is detected by a method comprising performing an amplification reaction that generates an amplicon corresponding to a portion of the E6/E7 region of the HPV genome and using a nucleic acid probe;
wherein the high-risk HPV is selected from the group consisting of HPV 16, HPV 18, and HPV 45, and wherein an HPV 16 primer and probe set, an HPV 18 primer and probe set, and/or an HPV 45 primer and probe set, is used in the amplification reaction for genotyping high-risk HPV, wherein:
i) the HPV 16 primer and probe set comprises:
(i) a primer capable of amplifying a portion of the E6/E7 region of the HPV 16 genome, but not an E6/E7 region of HPV 18 or HPV 45 genome, wherein the primer has 70% to 100% identity with a sequence of SEQ ID NO:1,
(ii) a primer capable of amplifying a portion of the E6/E7 region of the HPV 16 genome, but not an E6/E7 region of HPV 18 or HPV 45 genome, wherein the primer has 70% to 100% identity with a sequence of SEQ ID NO:2, and,
(iii) a probe having 70% to 100% identity with a sequence of SEQ ID NO:3;
ii) the HPV 18 primer and probe set comprises:
(i) a primer capable of amplifying a portion of the E6/E7 region of the HPV 18 genome, but not an E6/E7 region of HPV 16 or HPV 45 genome, wherein the primer has 70% to 100% identity with a sequence of SEQ ID NO:4,
(ii) a primer capable of amplifying a portion of the E6/E7 region of the HPV 18 genome, but not an E6/E7 region of HPV 16 or HPV 45 genome, wherein the primer has 70% to 100% identity with a sequence of SEQ ID NO:5, and,
(iii) a probe having 70% to 100% identity with a sequence of SEQ ID NO:6;
and
iii) the HPV 45 primer and probe set comprises:
(i) a primer capable of amplifying a portion of the E6/E7 region of the HPV 45 genome, but not an E6/E7 region of HPV 16 or HPV 18 genome, wherein the first primer has 70% to 100% identity with a sequence of SEQ ID NO:7,
(ii) a primer capable of amplifying a portion of the E6/E7 region of the HPV 45 genome, but not an E6/E7 region of HPV 16 or HPV 18 genome, wherein the primer has 70% to 100% identity with a sequence of SEQ ID NO:8, and,
(iii) a probe having 70% to 100% identity with a sequence of SEQ ID NO:9.

2. The method of claim 1, wherein the said method is performed in a real time multiplex PCR format.

3. The method of claim 1 or 2, wherein said method is capable of distinguishing between HPV 16, HPV 18, and HPV 45 infections, said method comprising:
a. providing a sample suspected of comprising a high-risk HPV;
b. contacting the sample with:
(i) a primer set capable of amplifying a portion of a target sequence of an E6/E7 region of the HPV 16 genome, but not an E6/E7 region of an HPV 18 or HPV 45 genome, wherein the primer set comprises a primer having 70% to 100% identity with a sequence of SEQ ID NO:1 and a primer having 70% to 100% identity with a sequence of SEQ ID NO:2;
(ii) a primer set capable of amplifying a portion of a target sequence of an E6/E7 region of the HPV 18 genome, but not an E6/E7 region of an HPV 16 or HPV 45 genome, wherein the primer set comprises a primer having 70% to 100% identity with a sequence of SEQ ID NO:4, and a primer having 70% to 100% identity with a sequence of SEQ ID NO:5;
(iii) a primer set capable of amplifying a portion of a target sequence of an E6/E7 region of the HPV 45 genome, but not an E6/E7 region of an HPV 16 or HPV 18 genome, wherein the primer set comprises a primer having 70% to 100% identity with a sequence of SEQ ID NO:7 and a primer having 70% to 100% identity with a sequence of SEQ ID NO:8;
(iv) a detectably labeled nucleic acid probe capable of hybridizing to a portion of the target sequence of the E6/E7 region of the HPV 16 genome, but not an E6/E7 region of an HPV 18 or HPV 45 genome, wherein the probe has 70% to 100% identity with a sequence of SEQ ID NO:3;
(v) a detectably labeled nucleic acid probe capable of hybridizing to a portion of the target sequence of the E6/E7 region of the HPV 18 genome, but not an E6/E7 region of an HPV 16 or HPV 45 genome, wherein the probe has 70% to 100% identity with a sequence of SEQ ID NO:6; and
(vi) a detectably labeled nucleic acid probe capable of hybridizing to a portion the target sequence of the E6/E7 region of the HPV 45 genome, but not an E6/E7 region of an HPV 16 or HPV 18 genome, wherein the probe has 70% to 100% identity with a sequence of SEQ ID NO:9;
wherein each nucleic acid probe has a different detectable label;
c. performing a nucleic acid amplification; and
d. detecting the presence or absence of each detectable label.

4. The method according to any of claims 1 to 3, comprising using a primer pair selected from the group consisting of:
a. a first primer consisting of SEQ ID NO: 1 and a second primer having about 70% to 100% identity SEQ ID NO: 2,
b. a first primer consisting of SEQ ID NO: 4 and a second primer having about 70% to 100% identity SEQ ID NO: 5, and
c. a first primer consisting of SEQ ID NO: 7 and a second primer having about 70% to 100% identity SEQ ID NO: 8.

5. The method according to any one of claims 1 to 4, further comprising using a control primer and probe set, which preferably has the following characteristics:
i) it is a beta globin control primer and probe set; or
ii) it is a beta globin control primer and probe set, wherein said control primer and probe set comprises
a. a primer having 70% to 100% identity with a sequence of SEQ ID NO: 16,
b. a primer having 70% to 100% identity with a sequence of SEQ ID NO: 17, and,
c. a probe having 70% to 100% identity with a sequence of SEQ ID NO: 18.

## Patentansprüche

1. Verfahren zur Genotypisierung eines Hochrisiko-HPV, das Verfahren umfassend:
a. Hybridisieren von mindestens einem Nukleinsäure-Primer an mindestens einen Teil einer Zielsequenz in einer E6/E7 Region eines ersten Hochrisiko-HPV Genoms, wobei der Nukleinsäure-Primer spezifisch für eine Zielsequenz in der E6/E7 Region eines Hochrisiko-HPV Genoms ist und wobei der Nukleinsäure-Primer aa) 70% oder mehr Übereinstimmung über die gesamte Länge zu sowohl der Zielsequenz und mindestens einer Nukleinsäuresequenz in der E6/E7 Region eines Genoms eines Subtyps davon aufweist und bb) der Nukleinsäure-Primer nicht an eine Nukleinsäure hybridisiert, die von einem anderen HPV Typ entstammt; und
b. Detektieren der Hybridisierung des Nukleinsäure-Primers an die E6/E7 Region des ersten Hochrisiko-HPV Genoms, wobei die Hybridisierung durch ein Verfahren detektiert wird, umfassend die Durchführung einer Amplifizierungsreaktion, die ein Amplicon generiert, das einem Teil der E6/E7 Region des HPV Genoms entspricht, und Einsatz einer Nukleinsäure-Sonde;
wobei der Hochrisiko-HPV ausgewählt wird aus der Gruppe bestehend aus HPV 16, HPV 18 und HPV 45 und wobei ein HPV 16 Primer und Sonden-Set, ein HPV 18 Primer und Sonden-Set und/oder ein HPV 45 Primer und Sonden-Set in der Amplifizierungsreaktion zur Hochrisiko-HPV Genotypisierung genutzt wird, wobei:
i) das HPV 16 Primer und Sonden-Set aufweist:
(i) einen Primer, der in der Lage ist, einen Teil der E6/E7 Region des HPV 16 Genoms zu amplifizieren, jedoch nicht eine E6/E7 Region eines HPV 18 oder HPV 45 Genoms, wobei der Primer 70% bis 100% Übereinstimmung mit einer Sequenz gemäß SEQ ID NO:1 aufweist,
(ii) einen Primer, der in der Lage ist, einen Teil der E6/E7 Region des HPV 16 Genoms zu amplifizieren, jedoch nicht eine E6/E7 Region eines HPV 18 oder HPV 45 Genoms, wobei der Primer 70% bis 100% Übereinstimmung mit einer Sequenz gemäß SEQ ID NO:2 aufweist, und
(iii) eine Sonde, die 70% bis 100% Übereinstimmung mit einer Sequenz gemäß SEQ ID NO:3 aufweist;
ii) das HPV 18 Primer und Sonden-Set aufweist:
(i) einen Primer, der in der Lage ist, einen Teil der E6/E7 Region des HPV 18 Genoms zu amplifizieren, jedoch nicht eine E6/E7 Region eines HPV 16 oder HPV 45 Genoms, wobei der Primer 70% bis 100% Übereinstimmung mit einer Sequenz gemäß SEQ ID NO:4 aufweist,
(ii) einen Primer, der in der Lage ist, einen Teil der E6/E7 Region des HPV 18 Genoms zu amplifizieren, jedoch nicht eine E6/E7 Region eines HPV 16 oder HPV 45 Genoms, wobei der Primer 70% bis 100% Übereinstimmung mit einer Sequenz gemäß SEQ ID NO:5 aufweist, und
(iii) eine Sonde, die 70% bis 100% Übereinstimmung mit einer Sequenz gemäß SEQ ID NO:6 aufweist;
iii) das HPV 45 Primer und Sonden-Set umfasst:
(i) einen Primer, der in der Lage ist, einen Teil der E6/E7 Region des HPV 45 Genoms zu amplifizieren, jedoch nicht eine E6/E7 Region eines HPV 16 oder HPV 18 Genoms, wobei der Primer 70% bis 100% Übereinstimmung mit einer Sequenz gemäß SEQ ID NO:7 aufweist,
(ii) einen Primer, der in der Lage ist, einen Teil der E6/E7 Region des HPV 45 Genoms zu amplifizieren, jedoch nicht eine E6/E7 Region eines HPV 16 oder HPV 18 Genoms, wobei der Primer 70% bis 100% Übereinstimmung mit einer Sequenz gemäß SEQ ID NO:8 aufweist, und
(iii) eine Sonde, die 70% bis 100% Übereinstimmung mit einer Sequenz gemäß SEQ ID NO:9 aufweist.

2. Das Verfahren nach Anspruch 1, wobei das Verfahren in einem real-time Multiplex-PCR Format durchgeführt wird.

3. Das Verfahren nach Anspruch 1 oder 2, wobei das Verfahren in der Lage ist, zwischen HPV 16, HPV 18 und HPV 45 Infektionen zu unterscheiden, das Verfahren umfassend:
a. Bereitstellen einer Probe, bei der vermutet wird, einen Hochrisiko-HPV aufzuweisen;
b. in Kontakt bringen der Probe mit:
(i) einem Primer-Set, das in der Lage ist, einen Teil einer Zielsequenz einer E6/E7 Region eines HPV 16 Genoms zu amplifizieren, jedoch nicht eine E6/E7 Region eines HPV 18 oder HPV 45 Genoms, wobei das Primer-Set einen Primer, der 70% bis 100% Übereinstimmung mit einer Sequenz gemäß SEQ ID NO:1 aufweist, und einen Primer, der 70% bis 100% Übereinstimmung mit einer Sequenz gemäß SEQ ID NO:2 aufweist, umfasst;
(ii) einem Primer-Set, das in der Lage ist, einen Teil der Zielsequenz einer E6/E7 Region eines HPV 18 Genoms zu amplifizieren, jedoch nicht eine E6/E7 Region eines HPV 16 oder HPV 45 Genoms, wobei das Primer-Set einen Primer, der 70% bis 100% Übereinstimmung mit einer Sequenz gemäß SEQ ID NO:4 aufweist, und einen Primer, der 70% bis 100% Übereinstimmung mit einer Sequenz gemäß SEQ ID NO:5 aufweist, umfasst;
(iii) einem Primer-Set, das in der Lage ist, einen Teil der Zielsequenz einer E6/E7 Region eines HPV 45 Genoms zu amplifizieren, jedoch nicht eine E6/E7 Region eines HPV 16 oder HPV 18 Genoms, wobei das Primer-Set einen Primer, der 70% bis 100% Übereinstimmung mit einer Sequenz gemäß SEQ ID NO:7 aufweist, und einen Primer, der 70% bis 100% Übereinstimmung mit einer Sequenz gemäß SEQ ID NO:8 aufweist, umfasst;
(iv) eine detektierbar markierte Nukleinsäuresonde, die in der Lage ist, an einen Teil einer Zielsequenz der E6/E7 Region des HPV 16 Genoms zu hybridisieren, jedoch nicht an eine E6/E7 Region des HPV 18 oder HPV 45 Genoms, wobei die Sonde 70% bis 100% Übereinstimmung mit einer Sequenz gemäß SEQ ID NO:3 aufweist;
(v) eine detektierbar markierte Nukleinsäuresonde, die in der Lage ist, an einen Teil einer Zielsequenz der E6/E7 Region des HPV 18 Genoms zu hybridisieren, jedoch nicht an eine E6/E7 Region des HPV 16 oder HPV 45 Genoms, wobei die Sonde 70% bis 100% Übereinstimmung mit einer Sequenz gemäß SEQ ID NO:6 aufweist; und
(vi) eine detektierbar markierte Nukleinsäuresonde, die in der Lage ist, an einen Teil einer Zielsequenz der E6/E7 Region des HPV 45 Genoms zu hybridisieren, jedoch nicht an eine E6/E7 Region des HPV 16 oder HPV 18 Genoms, wobei die Sonde 70% bis 100% Übereinstimmung mit einer Sequenz gemäß SEQ ID NO:9 aufweist;
wobei jede Nukleinsäuresonde eine andere, detektierbare Markierung aufweist;
c. Durchführen einer Nukleinsäure-Amplifikation;
d. Detektieren der Anwesenheit oder Abwesenheit von jeder detektierbaren Markierung.

4. Das Verfahren nach einem der Ansprüche 1 bis 3, umfassend den Einsatz eines Primer-Paars, das ausgewählt ist aus der Gruppe bestehend aus:
a. einem ersten Primer bestehend aus SEQ ID NO: 1 und einem zweiten Primer, der ungefähr 70% bis 100% Übereinstimmung mit SEQ ID NO: 2 aufweist,
b. einem ersten Primer bestehend aus SEQ ID NO: 4 und einem zweiten Primer, der ungefähr 70% bis 100% Übereinstimmung mit SEQ ID NO: 5 aufweist,
c. einem ersten Primer bestehend aus SEQ ID NO: 7 und einem zweiten Primer, der ungefähr 70% bis 100% Übereinstimmung mit SEQ ID NO: 8 aufweist.

5. Das Verfahren nach einem der Ansprüche 1 bis 4, weiter umfassend den Einsatz eines Kontroll-Primer und Sonden-Sets, das vorzugsweise folgende Eigenschaften hat:
i) es ist ein beta-Globin Kontroll-Primer und Sonden-Set; oder
ii) es ist ein beta-Globin Kontroll-Primer und Sonden-Set, wobei das Kontroll-Primer und Sonden-Set umfasst
a. einen Primer, der 70% bis 100% Übereinstimmung mit einer Sequenz gemäß SEQ ID NO: 16 aufweist,
b. einen Primer, der 70% bis 100% Übereinstimmung mit einer Sequenz gemäß SEQ ID NO: 17 aufweist, und
c. eine Sonde, die 70% bis 100% Übereinstimmung mit einer Sequenz gemäß SEQ ID NO: 18 aufweist.

## Revendications

1. Procédé de génotypage d'un HPV à haut risque, le procédé comprenant :
a. l'hybridation d'au moins une amorce d'acide nucléique avec au moins une partie d'une séquence cible dans une région E6/E7 d'un premier génome de HPV à haut risque, dans lequel l'amorce d'acide nucléique est spécifique pour une séquence cible dans la région E6/E7 d'un génome de HPV à haut risque et dans lequel ladite amorce d'acide nucléique présente aa) 70 % d'identité ou plus sur sa longueur totale à la fois avec la séquence cible et au moins une séquence d'acide nucléique dans la région E6/E7 d'un génome d'un sous-type de celui-ci et bb) ladite amorce d'acide nucléique ne s'hybride pas avec un acide nucléique dérivé d'un type de HPV différent ; et
b. la détection de l'hybridation de l'amorce d'acide nucléique avec la région E6/E7 du premier génome de HPV à haut risque, dans lequel l'hybridation est détectée par un procédé comprenant la conduite d'une réaction d'amplification qui génère un amplicon correspondant à une partie de la région E6/E7 du génome de HPV et l'utilisation d'une sonde d'acide nucléique ;
dans lequel le HPV à haut risque est choisi dans le groupe constitué de HPV 16, HPV 18, et HPV 45, et dans lequel un ensemble d'amorces et de sondes de HPV 16, un ensemble d'amorces et de sondes de HPV 18, et/ou un ensemble d'amorces et de sondes de HPV 45, est utilisé dans la réaction d'amplification pour le génotypage de HPV à haut risque, dans lequel :
i) l'ensemble d'amorces et de sondes de HPV 16 comprend :
(i) une amorce capable d'amplifier une partie de la région E6/E7 du génome de HPV 16, mais pas une région E6/E7 du génome de HPV 18 ou HPV 45, dans lequel l'amorce présente 70 % à 100 % d'identité avec une séquence de SEQ ID NO: 1,
(ii) une amorce capable d'amplifier une partie de la région E6/E7 du génome de HPV 16, mais pas une région E6/E7 du génome de HPV 18 ou HPV 45, dans lequel l'amorce présente 70 % à 100 % d'identité avec une séquence de SEQ ID NO: 2, et,
(iii) une sonde ayant 70 % à 100 % d'identité avec une séquence de SEQ ID NO: 3 ;
ii) l'ensemble d'amorces et de sondes de HPV 18 comprend :
(i) une amorce capable d'amplifier une partie de la région E6/E7 du génome de HPV 18, mais pas une région E6/E7 du génome de HPV 16 ou HPV 45, dans lequel l'amorce présente 70 % à 100 % d'identité avec une séquence de SEQ ID NO: 4,
(ii) une amorce capable d'amplifier une partie de la région E6/E7 du génome de HPV 18, mais pas une région E6/E7 du génome de HPV 16 ou HPV 45, dans lequel l'amorce présente 70 % à 100 % d'identité avec une séquence de SEQ ID NO: 5, et,
(iii) une sonde ayant 70 % à 100 % d'identité avec une séquence de SEQ ID NO: 6 ;
et
iii) l'ensemble d'amorces et de sondes de HPV 45 comprend :
(i) une amorce capable d'amplifier une partie de la région E6/E7 du génome de HPV 45, mais pas une région E6/E7 du génome de HPV 16 ou HPV 18, dans lequel la première amorce présente 70 % à 100 % d'identité avec une séquence de SEQ ID NO:7,
(ii) une amorce capable d'amplifier une partie de la région E6/E7 du génome de HPV 45, mais pas une région E6/E7 du génome de HPV 16 ou HPV 18, dans lequel l'amorce présente 70 % à 100 % d'identité avec une séquence de SEQ ID NO: 8, et,
(iii) une sonde ayant 70 % à 100 % d'identité avec une séquence de SEQ ID NO: 9.

2. Procédé selon la revendication 1, ledit procédé étant conduit dans un format de PCR multiplexe en temps réel.

3. Procédé selon la revendication 1 ou 2, ledit procédé étant capable de distinguer entre des infections par HPV 16, HPV 18 et HPV 45, ledit procédé comprenant :
a. la fourniture d'un échantillon suspecté de comprendre un HPV à haut risque ;
b. la mise en contact de l'échantillon avec :
(i) un ensemble d'amorces capable d'amplifier une partie d'une séquence cible d'une région E6/E7 du génome de HPV 16, mais pas une région E6/E7 d'un génome de HPV 18 ou HPV 45, dans lequel l'ensemble d'amorces comprend une amorce ayant 70 % à 100 % d'identité avec une séquence de SEQ ID NO: I et une amorce ayant 70 % à 100 % d'identité avec une séquence de SEQ ID NO: 2 ;
(ii) un ensemble d'amorces capable d'amplifier une partie d'une séquence cible d'une région E6/E7 du génome de HPV 18, mais pas une région E6/E7 d'un génome de HPV 16 ou HPV 45, dans lequel l'ensemble d'amorces comprend une amorce ayant 70 % à 100 % d'identité avec une séquence de SEQ ID NO: 4, et une amorce ayant 70 % à 100 % d'identité avec une séquence de SEQ ID NO: 5 ;
(iii) un ensemble d'amorces capable d'amplifier une partie d'une séquence cible d'une région E6/E7 du génome de HPV 45, mais pas une région E6/E7 d'un génome de HPV 16 ou HPV 18, dans lequel l'ensemble d'amorces comprend une amorce ayant 70 % à 100 % d'identité avec une séquence de SEQ ID NO: 7 et une amorce ayant 70 % à 100 % d'identité avec une séquence de SEQ ID NO: 8 ;
(iv) une sonde d'acide nucléique marquée de façon détectable capable de s'hybrider avec une partie de la séquence cible de la région E6/E7 du génome de HPV 16, mais pas une région E6/E7 d'un génome de HPV 18 ou HPV 45, dans lequel la sonde présente 70 % à 100 % d'identité avec une séquence de SEQ ID NO: 3 ;
(v) une sonde d'acide nucléique marquée de façon détectable capable de s'hybrider avec une partie de la séquence cible de la région E6/E7 du génome de HPV 18, mais pas une région E6/E7 d'un génome de HPV 16 ou HPV 45, dans lequel la sonde présente 70 % à 100 % d'identité avec une séquence de SEQ ID NO: 6 ; et
(vi) une sonde d'acide nucléique marquée de façon détectable capable de s'hybrider avec une partie de la séquence cible de la région E6/E7 du génome de HPV 45, mais pas une région E6/E7 d'un génome de HPV 16 ou HPV 18, dans lequel la sonde présente 70 % à 100 % d'identité avec une séquence de SEQ ID NO: 9 ;
dans lequel chaque sonde d'acide nucléique comporte un marqueur détectable différent ;
c. la conduite d'une amplification d'acide nucléique ; et
d. la détection de la présence ou l'absence de chaque marqueur détectable.

4. Procédé selon l'une quelconque des revendications 1 à 3, comprenant l'utilisation d'une paire d'amorces choisie dans le groupe constitué de :
a. une première amorce constituée de SEQ ID NO: 1 et une deuxième amorce ayant environ 70 % à 100 % d'identité avec SEQ ID NO: 2,
b. une première amorce constituée de SEQ ID NO: 4 et une deuxième amorce ayant environ 70 % à 100 % d'identité avec SEQ ID NO: 5, et
c. une première amorce constituée de SEQ ID NO: 7 et une deuxième amorce ayant environ 70 % à 100 % d'identité avec SEQ ID NO: 8.

5. Procédé selon l'une quelconque des revendications 1 à 4, comprenant en outre l'utilisation d'un ensemble d'amorces et de sondes témoin, qui présente de préférence les caractéristiques suivantes :
i) c'est une ensemble d'amorces et de sondes témoin de bêta-globine ; ou
ii) c'est une ensemble d'amorces et de sondes témoin de bêta-globine, dans lequel ledit ensemble d'amorces et de sondes témoin comprend
a. une amorce ayant 70 % à 100 % d'identité avec une séquence de SEQ ID NO: 16,
b. une amorce ayant 70 % à 100 % d'identité avec une séquence de SEQ ID NO: 17, et,
c. une sonde ayant 70 % à 100 % d'identité avec une séquence de SEQ ID NO: 18.


### SEQUENCE LISTING

<110> Rangwala, Sameera Kobayashi, Lori Gay, Tanya
<120> Multiplex Assays and Methods
<130> 2912943-076001
<150> 61/430,797
   <151> 2011-01-07
<160> 21
<170> PatentIn version 3.5
<210> 1
   <211> 24
   <212> DNA
   <213> Artificial Sequence
<220>
   <223> pcr primer
<400> 1
   agaaccggac agagcccatt acaa 24
<210> 2
   <211> 24
   <212> DNA
   <213> Artificial Sequence
<220>
   <223> pcr primer
<400> 2
   gcacacaatt cctagtgtgc ccat 24
<210> 3
   <211> 25
   <212> DNA
   <213> Artificial Sequence
<220>
   <223> pcr probe
<400> 3
   acgcttcggt tgtgcgtaca aagca 25
<210> 4
   <211> 24
   <212> DNA
   <213> Artificial Sequence
<220>
   <223> pcr primer
<400> 4
   tcatcaacat ttaccagccc gacg 24
<210> 5
   <211> 24
   <212> DNA
   <213> Artificial Sequence
<220>
   <223> pcr primer
<400> 5
   gaaacagctg ctggaatgct cgaa 24
<210> 6
   <211> 26
   <212> DNA
   <213> Artificial Sequence
<220>
   <223> pcr probe
<400> 6
   agccgaacca caacgtcaca caatgt 26
<210> 7
   <211> 24
   <212> DNA
   <213> Artificial Sequence
<220>
   <223> pcr primer
<400> 7
   ttgacgatcc aaagcaacga ccct 24
<210> 8
   <211> 24
   <212> DNA
   <213> Artificial Sequence
<220>
   <223> pcr primer
<400> 8
   cctctgtgcg ttccaatgtt gctt 24
<210> 9
   <211> 30
   <212> DNA
   <213> Artificial Sequence
<220>
   <223> pcr probe
<400> 9
   acaagctacc agatttgtgc acagaattga 30
<210> 10
   <211> 24
   <212> DNA
   <213> Artificial Sequence
<220>
   <223> pcr primer
<400> 10
   ttgttaataa ggtgcctgcg gtgc 24
<210> 11
   <211> 24
   <212> DNA
   <213> Artificial Sequence
<220>
   <223> pcr primer
<400> 11
   tgtttcccta cgtctgcgaa gtct 24
<210> 12
   <211> 26
   <212> DNA
   <213> Artificial Sequence
<220>
   <223> pcr probe
<400> 12
   atacatgttg tgaccaggca cggcaa 26
<210> 13
   <211> 7904
   <212> DNA
   <213> Human papillomavirus type 16
<400> 13
   actacaataa ttcatgtata aaactaaggg cgtaaccgaa atcggttgaa ccgaaaccgg      60
   ttagtataaa agcagacatt ttatgcacca aaagagaact gcaatgtttc aggacccaca     120
   ggagcgaccc agaaagttac cacagttatg cacagagctg caaacaacta tacatgatat     180
   aatattagaa tgtgtgtact gcaagcaaca gttactgcga cgtgaggtat atgactttgc     240
   ttttcgggat ttatgcatag tatatagaga tgggaatcca tatgctgtat gtgataaatg     300
   tttaaagttt tattctaaaa ttagtgagta tagacattat tgttatagtt tgtatggaac     360
   aacattagaa cagcaataca acaaaccgtt gtgtgatttg ttaattaggt gtattaactg     420
   tcaaaagcca ctgtgtcctg aagaaaagca aagacatctg gacaaaaagc aaagattcca     480
   taatataagg ggtcggtgga ccggtcgatg tatgtcttgt tgcagatcat caagaacacg     540
   tagagaaacc cagctgtaat catgcatgga gatacaccta cattgcatga atatatgtta     600
   gatttgcaac cagagacaac tgatctctac tgttatgagc aattaaatga cagctcagag     660
   gaggaggatg aaatagatgg tccagctgga caagcagaac cggacagagc ccattacaat      720
   attgtaacct tttgttgcaa gtgtgactct acgcttcggt tgtgcgtaca aagcacacac      780
   gtagacattc gtactttgga agacctgtta atgggcacac taggaattgt gtgccccatc      840
   tgttctcaga aaccataatc taccatggct gatcctgcag gtaccaatgg ggaagagggt      900
   acgggatgta atggatggtt ttatgtagag gctgtagtgg aaaaaaaaac aggggatgct      960
   atatcagatg acgagaacga aaatgacagt gatacaggtg aagatttggt agattttata     1020
   gtaaatgata atgattattt aacacaggca gaaacagaga cagcacatgc gttgtttact     1080
   gcacaggaag caaaacaaca tagagatgca gtacaggttc taaaacgaaa gtatttggta     1140
   gtccacttag tgatattagt ggatgtgtag acaataatat tagtcctaga ttaaaagcta     1200
   tatgtataga aaaacaaagt agagctgcaa aaaggagatt atttgaaagc gaagacagcg     1260
   ggtatggcaa tactgaagtg gaaactcagc agatgttaca ggtagaaggg cgccatgaga     1320
   ctgaaacacc atgtagtcag tatagtggtg gaagtggggg tggttgcagt cagtacagta     1380
   gtggaagtgg gggagagggt gttagtgaaa gacacactat atgccaaaca ccacttacaa     1440
   atattttaaa tgtactaaaa actagtaatg caaaggcagc aatgttagca aaatttaaag     1500
   agttatacgg ggtgagtttt tcagaattag taagaccatt taaaagtaat aaatcaacgt     1560
   gttgcgattg gtgtattgct gcatttggac ttacacccag tatagctgac agtataaaaa     1620
   cactattaca acaatattgt ttatatttac acattcaaag tttagcatgt tcatggggaa     1680
   tggttgtgtt actattagta agatataaat gtggaaaaaa tagagaaaca attgaaaaat     1740
   tgctgtctaa actattatgt gtgtctccaa tgtgtatgat gatagagcct ccaaaattgc     1800
   gtagtacagc agcagcatta tattggtata aaacaggtat atcaaatatt agtgaagtgt     1860
   atggagacac gccagaatgg atacaaagac aaacagtatt acaacatagt tttaatgatt     1920
   gtacatttga attatcacag atggtacaat gggcctacga taatgacata gtagacgata     1980
   gtgaaattgc atataaatat gcacaattgg cagacactaa tagtaatgca agtgcctttc     2040
   taaaaagtaa ttcacaggca aaaattgtaa aggattgtgc aacaatgtgt agacattata     2100
   aacgagcaga aaaaaaacaa atgagtatga gtcaatggat aaaatataga tgtgataggg     2160
   tagatgatgg aggtgattgg aagcaaattg ttatgttttt aaggtatcaa ggtgtagagt     2220
   ttatgtcatt tttaactgca ttaaaaagat ttttgcaagg catacctaaa aaaaattgca     2280
   tattactata tggtgcagct aacacaggta aatcattatt tggtatgagt ttaatgaaat     2340
   ttctgcaagg gtctgtaata tgttttgtaa attctaaaag ccatttttgg ttacaaccat     2400
   tagcagatgc caaaataggt atgttagatg atgctacagt gccctgttgg aactacatag     2460
   atgacaattt aagaaatgca ttggatggaa atttagtttc tatggatgta aagcatagac     2520
   cattggtaca actaaaatgc cctccattat taattacatc taacattaat gctggtacag     2580
   attctaggtg gccttattta cataatagat tggtggtgtt tacatttcct aatgagtttc 2640
   catttgacga aaacggaaat ccagtgtatg agcttaatga taagaactgg aaatcctttt 2700
   tctcaaggac gtggtccaga ttaagtttgc acgaggacga ggacaaggaa aacgatggag 2760
   actctttgcc aacgtttaaa tgtgtgtcag gacaaaatac taacacatta tgaaaatgat 2820
   agtacagacc tacgtgacca tatagactat tggaaacaca tgcgcctaga atgtgctatt 2880
   tattacaagg ccagagaaat gggatttaaa catattaacc accaagtggt gccaacactg 2940
   gctgtatcaa agaataaagc attacaagca attgaactgc aactaacgtt agaaacaata 3000
   tataactcac aatatagtaa tgaaaagtgg acattacaag acgttagcct tgaagtgtat 3060
   ttaactgcac caacaggatg tataaaaaaa catggatata cagtggaagt gcagtttgat 3120
   ggagacatat gcaatacaat gcattataca aactggacac atatatatat ttgtgaagaa 3180
   gcatcagtaa ctgtggtaga gggtcaagtt gactattatg gtttatatta tgttcatgaa 3240
   ggaatacgaa catattttgt gcagtttaaa gatgatgcag aaaaatatag taaaaataaa 3300
   gtatgggaag ttcatgcggg tggtcaggta atattatgtc ctacatctgt gtttagcagc 3360
   aacgaagtat cctctcctga aattattagg cagcacttgg ccaaccaccc cgccgcgacc 3420
   cataccaaag ccgtcgcctt gggcaccgaa gaaacacaga cgactatcca gcgaccaaga 3480
   tcagagccag acaccggaaa cccctgccac accactaagt tgttgcacag agactcagtg 3540
   gacagtgctc caatcctcac tgcatttaac agctcacaca aaggacggat taactgtaat 3600
   agtaacacta cacccatagt acatttaaaa ggtgatgcta atactttaaa atgtttaaga 3660
   tatagattta aaaagcattg tacattgtat actgcagtgt cgtctacatg gcattggaca 3720
   ggacataatg taaaacataa aagtgcaatt gttacactta catatgatag tgaatggcaa 3780
   cgtgaccaat ttttgtctca agttaaaata ccaaaaacta ttacagtgtc tactggattt 3840
   atgtctatat gacaaatctt gatactgcat ccacaacatt actggcgtgc tttttgcttt 3900
   gctttgtgtg cttttgtgtg tctgcctatt aatacgtccg ctgcttttgt ctgtgtctac 3960
   atacacatca ttaataatat tggtattact attgtggata acagcagcct ctgcgtttag 4020
   gtgttttatt gtatatatta tatttgttta tataccatta tttttaatac atacacatgc 4080
   acgcttttta attacataat gtatatgtac ataatgtaat tgttacatat aattgttgta 4140
   taccataact tactattttt tcttttttat tttcatatat aatttttttt tttgtttgtt 4200
   tgtttgtttt ttaataaact gttattactt aacaatgcga cacaaacgtt ctgcaaaacg 4260
   cacaaaacgt gcatcggcta cccaacttta taaaacatgc aaacaggcag gtacatgtcc 4320
   acctgacatt atacctaagg ttgaaggcaa aactattgct gaacaaatat tacaatatgg 4380
   aagtatgggt gtattttttg gtgggttagg aattggaaca gggtcgggta caggcggacg 4440
   cactgggtat attccattgg gaacaaggcc tcccacagct acagatacac ttgctcctgt 4500
   aagaccccct ttaacagtag atcctgtggg cccttctgat ccttctatag tttctttagt 4560
   ggaagaaact agttttattg atgctggtgc accaacatct gtaccttcca ttcccccaga 4620
   tgtatcagga tttagtatta ctacttcaac tgataccaca cctgctatat tagatattaa 4680
   taatactgtt actactgtta ctacacataa taatcccact ttcactgacc catctgtatt 4740
   gcagcctcca acacctgcag aaactggagg gcattttaca ctttcatcat ccactattag 4800
   tacacataat tatgaagaaa ttcctatgga tacatttatt gttagcacaa accctaacac 4860
   agtaactagt agcacaccca taccagggtc tcgcccagtg gcacgcctag gattatatag 4920
   tcgcacaaca caacaggtta aagttgtaga ccctgctttt gtaaccactc ccactaaact 4980
   tattacatat gataatcctg catatgaagg tatagatgtg gataatacat tatatttttc 5040
   tagtaatgat aatagtatta atatagctcc agatcctgac tttttggata tagttgcttt 5100
   acataggcca gcattaacct ctaggcgtac tggcattagg tacagtagaa ttggtaataa 5160
   acaaacacta cgtactcgta gtggaaaatc tataggtgct aaggtacatt attattatga 5220
   tttaagtact attgatcctg cagaagaaat agaattacaa actataacac cttctacata 5280
   tactaccact tcacatgcag cctcacctac ttctattaat aatggattat atgatattta 5340
   tgcagatgac tttattacag atacttctac aaccccggta ccatctgtac cctctacatc 5400
   tttatcaggt tatattcctg caaatacaac aattcctttt ggtggtgcat acaatattcc 5460
   tttagtatca ggtcctgata tacccattaa tataactgac caagctcctt cattaattcc 5520
   tatagttcca gggtctccac aatatacaat tattgctgat gcaggtgact tttatttaca 5580
   tcctagttat tacatgttac gaaaacgacg taaacgttta ccatattttt tttcagatgt 5640
   ctctttggct gcctagtgag gccactgtct acttgcctcc tgtcccagta tctaaggttg 5700
   taagcacgga tgaatatgtt gcacgcacaa acatatatta tcatgcagga acatccagac 5760
   tacttgcagt tggacatccc tattttccta ttaaaaaacc taacaataac aaaatattag 5820
   ttcctaaagt atcaggatta caatacaggg tatttagaat acatttacct gaccccaata 5880
   agtttggttt tcctgacacc tcattttata atccagatac acagcggctg gtttgggcct 5940
   gtgtaggtgt tgaggtaggt cgtggtcagc cattaggtgt gggcattagt ggccatcctt 6000
   tattaaataa attggatgac acagaaaatg ctagtgctta tgcagcaaat gcaggtgtgg 6060
   ataatagaga atgtatatct atggattaca aacaaacaca attgtgttta attggttgca 6120
   aaccacctat aggggaacac tggggcaaag gatccccatg taccaatgtt gcagtaaatc 6180
   caggtgattg tccaccatta gagttaataa acacagttat tcaggatggt gatatggttc 6240
   atactggctt tggtgctatg gactttacta cattacaggc taacaaaagt gaagttccac 6300
   tggatatttg tacatctatt tgcaaatatc cagattatat taaaatggtg tcagaaccat 6360
   atggcgacag cttatttttt tatttacgaa gggaacaaat gtttgttaga catttattta     6420
   atagggctgg tactgttggt gaaaatgtac cagacgattt atacattaaa ggctctgggt     6480
   ctactgcaaa tttagccagt tcaaattatt ttcctacacc tagtggttct atggttacct     6540
   ctgatgccca aatattcaat aaaccttatt ggttacaacg agcacagggc cacaataatg     6600
   gcatttgttg gggtaaccaa ctatttgtta ctgttgttga tactacacgc agtacaaata     6660
   tgtcattatg tgctgccata tctacttcag aaactacata taaaaatact aactttaagg     6720
   agtacctacg acatggggag gaatatgatt tacagtttat ttttcaactg tgcaaaataa     6780
   ccttaactgc agacgttatg acatacatac attctatgaa ttccactatt ttggaggact     6840
   ggaattttgg tctacaacct cccccaggag gcacactaga agatacttat aggtttgtaa     6900
   cccaggcaat tgcttgtcaa aaacatacac ctccagcacc taaagaagat gatcccctta     6960
   aaaaatacac tttttgggaa gtaaatttaa aggaaaagtt ttctgcagac ctagatcagt     7020
   ttcctttagg acgcaaattt ttactacaag caggattgaa ggccaaacca aaatttacat     7080
   taggaaaacg aaaagctaca cccaccacct catctacctc tacaactgct aaacgcaaaa     7140
   aacgtaagct gtaagtattg tatgtatgtt gaattagtgt tgtttgttgt gtatatgttt     7200
   gtatgtgctt gtatgtgctt gtaaatatta agttgtatgt gtgtttgtat gtatggtata     7260
   ataaacacgt gtgtatgtgt ttttaaatgc ttgtgtaact attgtgtcat gcaacataaa     7320
   taaacttatt gtttcaacac ctactaattg tgttgtggtt attcattgta tataaactat     7380
   atttgctaca tcctgttttt gttttatata tactatattt tgtagcgcca ggcccatttt     7440
   gtagcttcaa ccgaattcgg ttgcatgctt tttggcacaa aatgtgtttt tttaaatagt     7500
   tctatgtcag caactatggt ttaaacttgt acgtttcctg cttgccatgc gtgccaaatc     7560
   cctgttttcc tgacctgcac tgcttgccaa ccattccatt gttttttaca ctgcactatg     7620
   tgcaactact gaatcactat gtacattgtg tcatataaaa taaatcacta tgcgccaacg     7680
   ccttacatac cgctgttagg cacatatttt tggcttgttt taactaacct aattgcatat     7740
   ttggcataag gtttaaactt ctaaggccaa ctaaatgtca ccctagttca tacatgaact     7800
   gtgtaaaggt tagtcataca ttgttcattt gtaaaactgc acatgggtgt gtgcaaaccg     7860
   attttgggtt acacatttac aagcaactta tataataata ctaa                       7904
<210> 14
   <211> 7857
   <212> DNA
   <213> Human papillomavirus type 18
<400> 14
   attaatactt ttaacaattg tagtatataa aaaagggagt aaccgaaaac ggtcgggacc 60
   gaaaacggtg tatataaaag atgtgagaaa cacaccacaa tactatggcg cgctttgagg 120
   atccaacacg gcgaccctac aagctacctg atctgtgcac ggaactgaac acttcactgc     180
   aagacataga aataacctgt gtatattgca agacagtatt ggaacttaca gaggtatttg     240
   aatttgcatt taaagattta tttgtggtgt atagagacag tataccgcat gctgcatgcc     300
   ataaatgtat agatttttat tctagaatta gagaattaag acattattca gactctgtgt     360
   atggagacac attggaaaaa ctaactaaca ctgggttata caatttatta ataaggtgcc     420
   tgcggtgcca gaaaccgttg aatccagcag aaaaacttag acaccttaat gaaaaacgac     480
   gatttcacaa catagctggg cactatagag gccagtgcca ttcgtgctgc aaccgagcac     540
   gacaggaacg actccaacga cgcagagaaa cacaagtata atattaagta tgcatggacc     600
   taaggcaaca ttgcaagaca ttgtattgca tttagagccc caaaatgaaa ttccggttga     660
   ccttctatgt cacgagcaat taagcgactc agaggaagaa aacgatgaaa tagatggagt     720
   taatcatcaa catttaccag cccgacgagc cgaaccacaa cgtcacacaa tgttgtgtat     780
   gtgttgtaag tgtgaagcca gaattgagct agtagtagaa agctcagcag acgaccttcg     840
   agcattccag cagctgtttc tgaacaccct gtcctttgtg tgtccgtggt gtgcatccca     900
   gcagtaagca acaatggctg atccagaagg tacagacggg gagggcacgg gttgtaacgg     960
   ctggttttat gtacaagcta ttgtagacaa aaaaacagga gatgtaatat cagatgacga    1020
   ggacgaaaat gcaacagaca cagggtcgga tatggtagat tttattgata cacaaggaac    1080
   attttgtgaa caggcagagc tagagacagc acaggcattg ttccatgcgc aggaggtcca    1140
   caatgatgca caagtgttgc atgttttaaa acgaaagttt gcaggaggca gcacagaaaa    1200
   cagtccatta ggggagcggc tggaggtgga tacagagtta agtccacggt tacaagaaat    1260
   atctttaaat agtgggcaga aaaaggcaaa aaggcggctg tttacaatat cagatagtgg    1320
   ctatggctgt tctgaagtgg aagcaacaca gattcaggta actacaaatg gcgaacatgg    1380
   cggcaatgta tgtagtggcg gcagtacgga ggctatagac aacgggggca cagagggcaa    1440
   caacagcagt gtagacggta caagtgacaa tagcaatata gaaaatgtaa atccacaatg    1500
   taccatagca caattaaaag acttgttaaa agtaaacaat aaacaaggag ctatgttagc    1560
   agtatttaaa gacacatatg ggctatcatt tacagattta gttagaaatt ttaaaagtga    1620
   taaaaccacg tgtacagatt gggttacagc tatatttgga gtaaacccaa caatagcaga    1680
   aggatttaaa acactaatac agccatttat attatatgcc catattcaat gtctagactg    1740
   taaatgggga gtattaatat tagccctgtt gcgttacaaa tgtggtaaga gtagactaac    1800
   agttgctaaa ggtttaagta cgttgttaca cgtacctgaa acttgtatgt taattcaacc    1860
   accaaaattg cgaagtagtg ttgcagcact atattggtat agaacaggaa tatcaaatat    1920
   tagtgaagta atgggagaca cacctgagtg gatacaaaga cttactatta tacaacatgg    1980
   aatagatgat agcaattttg atttgtcaga aatggtacaa tgggcatttg ataatgagct 2040
   gacagatgaa agcgatatgg catttgaata tgccttatta gcagacagca acagcaatgc 2100
   agctgccttt ttaaaaagca attgccaagc taaatattta aaagattgtg ccacaatgtg 2160
   caaacattat aggcgagccc aaaaacgaca aatgaatatg tcacagtgga tacgatttag 2220
   atgttcaaaa atagatgaag ggggagattg gagaccaata gtgcaattcc tgcgatacca 2280
   acaaatagag tttataacat ttttaggagc cttaaaatca tttttaaaag gaacccccaa 2340
   aaaaaattgt ttagtatttt gtggaccagc aaatacagga aaatcatatt ttggaatgag 2400
   ttttatacac tttatacaag gagcagtaat atcatttgtg aattccacta gtcatttttg 2460
   gttggaaccg ttaacagata ctaaggtggc catgttagat gatgcaacga ccacgtgttg 2520
   gacatacttt gatacctata tgagaaatgc gttagatggc aatccaataa gtattgatag 2580
   aaagcacaaa ccattaatac aactaaaatg tcctccaata ctactaacca caaatataca 2640
   tccagcaaag gataatagat ggccatattt agaaagtaga ataacagtat ttgaatttcc 2700
   aaatgcattt ccatttgata aaaatggcaa tccagtatat gaaataaatg acaaaaattg 2760
   gaaatgtttt tttgaaagga catggtccag attagatttg cacgaggaag aggaagatgc 2820
   agacaccgaa ggaaaccctt tcggaacgtt taagtgcgtt gcaggacaaa atcatagacc 2880
   actatgaaaa tgacagtaaa gacatagaca gccaaataca gtattggcaa ctaatacgtt 2940
   gggaaaatgc aatattcttt gcagcaaggg aacatggcat acagacatta aaccaccagg 3000
   tggtgccagc ctataacatt tcaaaaagta aagcacataa agctattgaa ctgcaaatgg 3060
   ccctacaagg ccttgcacaa agtgcataca aaaccgagga ttggacactg caagacacat 3120
   gcgaggaact atggaataca gaacctactc actgctttaa aaaaggtggc caaacagtac 3180
   aagtatattt tgatggcaac aaagacaatt gtatgaccta tgtagcatgg gacagtgtgt 3240
   attatatgac tgatgcagga acatgggaca aaacggctac ctgtgtaagt cacaggggat 3300
   tgtattatgt aaaggaaggg tacaacacgt tttatataga atttaaaagt gaatgtgaaa 3360
   aatatgggaa cacaggtacg tgggaagtac attttgggaa taatgtaatt gattgtaatg 3420
   actctatgtg cagtaccagt gacgacacgg tatccgctac tcagcttgtt aaacagctac 3480
   agcacacccc ctcaccgtat tccagcaccg tgtccgtggg caccgcaaag acctacggcc 3540
   agacgtcggc tgctacacga cctggacact gtggactcgc ggagaagcag cattgtggac 3600
   ctgtcaaccc acttctcggt gcagctacac ctacaggcaa caacaaaaga cggaaactct 3660
   gtagtggtaa cactacgcct ataatacatt taaaaggtga cagaaacagt ttaaaatgtt 3720
   tacggtacag attgcgaaaa catagcgacc actatagaga tatatcatcc acctggcatt 3780
   ggacaggtgc aggcaatgaa aaaacaggaa tactgactgt aacataccat agtgaaacac 3840
   aaagaacaaa atttttaaat actgttgcaa ttccagatag tgtacaaata ttggtgggat 3900
   acatgacaat gtaatacata tgctgtagta ccaatatgtt atcacttatt tttttatttt     3960
   gcttttgtgt atgcatgtat gtgtgctgcc atgtcccgct tttgccatct gtctgtatgt     4020
   gtgcgtatgc atgggtattg gtatttgtgt atattgtggt aataacgtcc cctgccacag     4080
   cattcacagt atatgtattt tgttttttat tgcccatgtt actattgcat atacatgcta     4140
   tattgtcttt acagtaattg tataggttgt tttatacagt gtattgtaca ttgtatattt     4200
   tgttttatac cttttatgct ttttgtattt ttgtaataaa agtatggtat cccaccgtgc     4260
   cgcacgacgc aaacgggctt cggtaactga cttatataaa acatgtaaac aatctggtac     4320
   atgtccacct gatgttgttc ctaaggtgga gggcaccacg ttagcagata aaatattgca     4380
   atggtcaagc cttggtatat ttttgggtgg acttggcata ggtactggca gtggtacagg     4440
   gggtcgtaca gggtacattc cattgggtgg gcgttccaat acagtggtgg atgttggtcc     4500
   tacacgtccc ccagtggtta ttgaacctgt gggccccaca gacccatcta ttgttacatt     4560
   aatagaggac tccagtgtgg ttacatcagg tgcacctagg cctacgttta ctggcacgtc     4620
   tgggtttgat ataacatctg cgggtacaac tacacctgcg gttttggata tcacaccttc     4680
   gtctacctct gtgtctattt ccacaaccaa ttttaccaat cctgcatttt ctgatccgtc     4740
   cattattgaa gttccacaaa ctggggaggt ggcaggtaat gtatttgttg gtacccctac     4800
   atctggaaca catgggtatg aggaaatacc tttacaaaca tttgcttctt ctggtacggg     4860
   ggaggaaccc attagtagta ccccattgcc tactgtgcgg cgtgtagcag gtccccgcct     4920
   ttacagtagg gcctaccaac aagtgtcagt ggctaaccct gagtttctta cacgtccatc     4980
   ctctttaatt acatatgaca acccggcctt tgagcctgtg gacactacat taacatttga     5040
   tcctcgtagt gatgttcctg attcagattt tatggatatt atccgtctac ataggcctgc     5100
   tttaacatcc aggcgtggga ctgttcgctt tagtagatta ggtcaacggg caactatgtt     5160
   tacccgcagc ggtacacaaa taggtgctag ggttcacttt tatcatgata taagtcctat     5220
   tgcaccttcc ccagaatata ttgaactgca gcctttagta tctgccacgg aggacaatga     5280
   cttgtttgat atatatgcag atgacatgga ccctgcagtg cctgtaccat cgcgttctac     5340
   tacctccttt gcatttttta aatattcgcc cactatatct tctgcctctt cctatagtaa     5400
   tgtaacggtc cctttaacct cctcttggga tgtgcctgta tacacgggtc ctgatattac     5460
   attaccatct actacctctg tatggcccat tgtatcaccc acggcccctg cctctacaca     5520
   gtatattggt atacatggta cacattatta tttgtggcca ttatattatt ttattcctaa     5580
   gaaacgtaaa cgtgttccct atttttttgc agatggcttt gtggcggcct agtgacaata     5640
   ccgtatatct tccacctcct tctgtggcaa gagttgtaaa taccgatgat tatgtgactc     5700
   gcacaagcat attttatcat gctggcagct ctagattatt aactgttggt aatccatatt     5760
   ttagggttcc tgcaggtggt ggcaataagc aggatattcc taaggtttct gcataccaat 5820
   atagagtatt tagggtgcag ttacctgacc caaataaatt tggtttacct gatactagta 5880
   tttataatcc tgaaacacaa cgtttagtgt gggcctgtgc tggagtggaa attggccgtg 5940
   gtcagccttt aggtgttggc cttagtgggc atccatttta taataaatta gatgacactg 6000
   aaagttccca tgccgccacg tctaatgttt ctgaggacgt tagggacaat gtgtctgtag 6060
   attataagca gacacagtta tgtattttgg gctgtgcccc tgctattggg gaacactggg 6120
   ctaaaggcac tgcttgtaaa tcgcgtcctt tatcacaggg cgattgcccc cctttagaac 6180
   ttaaaaacac agttttggaa gatggtgata tggtagatac tggatatggt gccatggact 6240
   ttagtacatt gcaagatact aaatgtgagg taccattgga tatttgtcag tctatttgta 6300
   aatatcctga ttatttacaa atgtctgcag atccttatgg ggattccatg tttttttgct 6360
   tacggcgtga gcagcttttt gctaggcatt tttggaatag agcaggtact atgggtgaca 6420
   ctgtgcctca atccttatat attaaaggca caggtatgcg tgcttcacct ggcagctgtg 6480
   tgtattctcc ctctccaagt ggctctattg ttacctctga ctcccagttg tttaataaac 6540
   catattggtt acataaggca cagggtcata acaatggtgt ttgctggcat aatcaattat 6600
   ttgttactgt ggtagatacc actcgcagta ccaatttaac aatatgtgct tctacacagt 6660
   ctcctgtacc tgggcaatat gatgctacca aatttaagca gtatagcaga catgttgagg 6720
   aatatgattt gcagtttatt tttcagttgt gtactattac tttaactgca gatgttatgt 6780
   cctatattca tagtatgaat agcagtattt tagaggattg gaactttggt gttccccccc 6840
   cgccaactac tagtttggtg gatacatatc gttttgtaca atctgttgct attacctgtc 6900
   aaaaggatgc tgcaccggct gaaaataagg atccctatga taagttaaag ttttggaatg 6960
   tggatttaaa ggaaaagttt tctttagact tagatcaata tccccttgga cgtaaatttt 7020
   tggttcaggc tggattgcgt cgcaagccca ccataggccc tcgcaaacgt tctgctccat 7080
   ctgccactac gtcttctaaa cctgccaagc gtgtgcgtgt acgtgccagg aagtaatatg 7140
   tgtgtgtgta tatatatata catctattgt tgtgtttgta tgtcctgtgt ttgtgtttgt 7200
   tgtatgattg cattgtatgg tatgtatggt tgttgttgta tgttgtatgt tactatattt 7260
   gttggtatgt ggcattaaat aaaatatgtt ttgtggttct gtgtgttatg tggttgcgcc 7320
   ctagtgagta acaactgtat ttgtgtttgt ggtatgggtg ttgcttgttg ggctatatat 7380
   tgtcctgtat ttcaagttat aaaactgcac accttacagc atccatttta tcctacaatc 7440
   ctccattttg ctgtgcaacc gatttcggtt gcctttggct tatgtctgtg gttttctgca 7500
   caatacagta cgctggcact attgcaaact ttaatctttt gggcactgct cctacatatt 7560
   ttgaacaatt ggcgcgcctc tttggcgcat ataaggcgca cctggtatta gtcattttcc 7620
   tgtccaggtg cgctacaaca attgcttgca taactatatc cactccctaa gtaataaaac 7680
   tgcttttagg cacatatttt agtttgtttt tacttaagct aattgcatac ttggcttgta 7740
   caactacttt catgtccaac attctgtcta cccttaacat gaactataat atgactaagc 7800
   tgtgcataca tagtttatgc aaccgaaata ggttgggcag cacatactat acttttc 7857
<210> 15
   <211> 7858
   <212> DNA
   <213> Human papillomavirus type 45
<400> 15
   aatactttta acaattatac tacataaaaa agggtgtaac cgaaaacggt tgcaaccaaa 60
   aacggtgcat ataaaagctt tgtggaaaag tgcattacag gatggcgcgc tttgacgatc 120
   caaagcaacg accctacaag ctaccagatt tgtgcacaga attgaataca tcactacaag 180
   acgtatctat tgcctgtgta tattgcaaag caacattgga acgcacagag gtatatcaat 240
   ttgcttttaa agatttatgt atagtgtata gagactgtat agcatatgct gcatgccata 300
   aatgtataga cttttattcc agaattagag aattaagata ttattcaaac tctgtatatg 360
   gagagacact ggaaaaaata actaatacag agttgtataa tttgttaata aggtgcctgc 420
   ggtgccagaa accattgaac ccagcagaaa aacgtagaca ccttaaggac aaacgaagat 480
   ttcacagcat agctggacag taccgagggc agtgtaatac atgttgtgac caggcacggc 540
   aagaaagact tcgcagacgt agggaaacac aagtatagca ataagtatgc atggaccccg 600
   ggaaacactg caagaaattg tattgcattt ggaacctcag aatgaattag atcctgttga 660
   cctgttgtgt tacgagcaat taagcgagtc agaggaggaa aacgatgaag cagatggagt 720
   tagtcatgca caactaccag cccgacgagc cgaaccacag cgtcacaaaa ttttgtgtgt 780
   atgttgtaag tgtgacggca gaattgagct tacagtagag agctcggcag aggaccttag 840
   aacactacag cagctgtttt tgagcacctt gtcctttgtg tgtccgtggt gtgcaactaa 900
   ccaataatct acaatggcgg atccagaagg taccgacggg gagggaacgg ggtgtaatgg 960
   ctggttcttt gtagaaacaa ttgtagagaa aaaaacaggg gatgtaatat cagatgatga 1020
   ggatgaaact gcaacagata cagggtcgga tatggtagat tttattgaca cacaattatc 1080
   catttgtgaa caggcagagc aagagacagc acaggcattg ttccatgcgc aggaagttca 1140
   gaatgatgca caggtgttgc atcttttaaa acgaaagttt gcaggaggca gcaaggaaaa 1200
   cagtccatta ggggagcagc taagtgtgga tacggatcta agtccacggt tacaagaaat 1260
   ttcattaaat agtgggcaca aaaaagcaaa acgacggttg tttacaatat cagatagtgg 1320
   ctatggctgt tctgaagtgg aagctgcaga gactcaggta actgtaaaca ctaatgcgga 1380
   aaatggcggc agtgtacata gtacacaaag tagtggtggg gatagtagtg acaatgcaga 1440
   aaatgtagat ccgcattgca gtattacaga actaaaggag ctattacaag caagtaacaa 1500
   aaaggctgca atgctggcag tatttaaaga catatatggg ctgtcattta cggatttggt 1560
   tagaaatttt aaaagtgata aaacaacatg tacagattgg gtaatggcta tatttggagt 1620
   taatccaacg gtagcagaag gctttaaaac attaattaaa ccagcaacgt tatacgccca 1680
   tatccaatgt ttagattgta aatggggagt attaatatta gctttattaa gatataaatg 1740
   tggcaaaaat agactaactg ttgcaaaagg cttaagcaca ttgttgcacg tacctgaaac 1800
   atgtatgtta attgaaccac caaaattgcg aagtagtgtt gcagcattat actggtatag 1860
   aacaggtata tccaatatta gtgaagtaag tggagacaca cctgagtgga tacaaagact 1920
   gacaattatt caacatggta ttgacgatag taattttgat ttgtcagaca tggtgcaatg 1980
   ggcatttgat aatgacctta cagatgaaag tgatatggca tttcaatatg cccaattagc 2040
   agactgcaac agtaatgcag ctgcattttt aaaaagtaac tgccaagcca aatatttaaa 2100
   agattgtgct gtaatgtgta gacattataa aagagcacaa aaacgccaaa tgaatatgtc 2160
   tcaatggatt aaatatagat gttccaaaat agatgaaggt ggggattgga gacccatagt 2220
   acaattccta agatatcagg gagtagaatt tattagcttt ttaagggcac taaaggaatt 2280
   tcttaaagga acaccaaaaa aaaattgtat actgttatat ggacctgcaa atacaggaaa 2340
   atcgtatttt ggaatgagtt ttatacattt cctacaaggt gcaataatat catttgtaaa 2400
   ttcaaacagc catttttggt tagaaccgtt agcagatact aaggtagcca tgttggatga 2460
   tgccacacac acgtgttgga catattttga taattatatg agaaatgcat tagatggtaa 2520
   tcctataagt atagacagaa agcataaacc attattacag ctaaaatgtc ctccaatcct 2580
   attaacatcc aatattgatc cagcaaaaga taataaatgg ccatatttag aaagtagggt 2640
   gacggtattt acatttccac atgcatttcc atttgataaa aatggtaatc cagtatatga 2700
   aataaatgat aaaaattgga aatgtttttt tgaaaggaca tggtccagat tagatttgca 2760
   cgaggacgat gaagatgcag acaccgaagg aatccctttc ggaacgttta agtgcgttac 2820
   aggacaaaat actagaccac tatgaaaatg acagtaaaga cataaacagc caaataagtt 2880
   attggcaact tatacgtttg gaaaatgcaa tactatttac agcaagggaa catggtatta 2940
   ccaaactaaa ccaccaggtg gtgcctccta ttaacatttc aaaaagcaaa gcacataaag 3000
   ctattgaact gcaaatggcc ttaaagggcc ttgcacaaag caagtataac aatgaggaat 3060
   ggacactgca agatacatgc gaggaactat ggaatacaga accgtcgcag tgttttaaaa 3120
   aaggcggtaa aaccgtgcac gtatactttg atggcaacaa ggacaactgt atgaactatg 3180
   tagtatggga cagtatatat tatataactg agacagggat atgggacaaa acagcagcat 3240
   gtgttagcta ttggggtgta tattatataa aagatggaga taccacatat tatgtacaat 3300
   ttaaaagcga atgtgagaaa tatggaaata gtaatacgtg ggaagtacaa tatgggggca 3360
   atgtaattga ttgtaatgac tctatgtgca gtaccagtga cgacacggta tccgctactc 3420
   agattgttag acagctacaa cacgcctcca cgtcgacccc caaaaccgca tccgtgggca 3480
   ccccaaaacc ccacatccag acgccggcta ctaagcgacc tagacagtgt ggactcacag 3540
   agcagcacca cggacgtgtc aacacccacg tgcacaaccc gctcctgtgt tcaagtacaa 3600
   gtaacaacaa aagaaggaaa gtgtgtagtg gtaacactac gcctataata cacttaaaag 3660
   gtgacaaaaa cagtttgaaa tgtttaagat ataggctacg caaatatgca gaccattact 3720
   cagaaatatc ctccacctgg cattggacag gttgtaataa aaacactggt atattaactg 3780
   taacatataa tagtgaggta caaagaaata cctttttgga tgtagttact attcctaaca 3840
   gtgtacaaat ctcggtggga tacatgacta tatgaatctg tatattgtat acagtatgta 3900
   acattactat gctatcttta gtgtttttat tgtgcttttc tgtgtgcctt tatgtgtgct 3960
   gcaatgtccc gcttgtgcag tctgtctatg tgtgtgcttt tgcttggttg ttggtgtttc 4020
   tttttatagt tgttattaca tccccattaa cagcatttgc tgtatacatt tgttgctatt 4080
   tactacctat gtttgtatta catatgcatg ctttacacac catacaataa ttactataat 4140
   gtacagtaca gtgtaacata cctgtgatgt gcatgttgtt gtatttttgt atttttgtat 4200
   ttttgtattt ttgtatttta tatgtttaat aaaccatggt atcccaccgt gcagcacgtc 4260
   gcaagcgggc ctctgcaact gacttatata gaacatgtaa gcaatccggt acgtgccccc 4320
   ctgatgttat taacaaagtg gaaggcacaa ccttagctga taaaatttta cagtggtcta 4380
   gccttgggat atttttgggt ggccttggca ttggtaccgg cagtggttct ggaggccgta 4440
   cgggctatgt acccttaggg ggcaggtcta atactgttgt ggatgttggc cccactaggc 4500
   cacctgtggt tattgaacct gtagggccta ctgatccatc tattgttacg ttggtagagg 4560
   attccagtgt tgttgcctct ggtgctccgg ttcccacatt taccggaacc tctgggtttg 4620
   aaattacgtc ttctggtact accacaccag ctgtgttgga catcacacct accgtggact 4680
   ctgtttctat ttcgtcaact agttttacaa atcctgcatt ttctgatccc tctattattg 4740
   aggtgcccca aacaggggag gtatcaggta atatatttgt tggtacacca acatcgggca 4800
   gccatggata tgaggaaata cctttacaaa catttgcatc ttctgggtca ggtacggaac 4860
   ccattagtag tacccccctc cctactgtgc ggcgggtacg gggtccccgc ctgtatagta 4920
   gggctaatca acaggtccgt gtgtccacct cacagttttt aacacatccc tcatcgttgg 4980
   ttacatttga taatccagct tatgagcccc tggacaccac actatccttt gagcctacca 5040
   gtaatgttcc tgattccgat tttatggata ttattcgttt gcataggcca gcattatcct 5100
   ctagacgtgg cactgttaga tttagtagat tgggtcaaag ggcaaccatg tttacacgta 5160
   gtggtaaaca aatagggggt agggtacatt tttaccatga tataagcccc attgctgcta 5220
   cagaggaaat tgaattgcag cctttaatta gtgctacaaa tgatagtgac ctgtttgatg 5280
   tatatgcaga cttcccacct cctgcgtcca ctacacctag cactatacac aaatcattta     5340
   catatccaaa gtattccttg accatgcctt ctactgctgc atcctcttac agtaatgtta     5400
   cagtaccatt aacatctgca tgggatgtac ctatatatac tggcccggac attatattgc     5460
   catcccatac tcctatgtgg cctagtacat ctcctaccaa tgcttccacc accacctata     5520
   taggtattca tggcacacaa tattatttat ggccatggta ttattatttt cctaaaaaac     5580
   gtaaacgtat tccctatttt tttgcagatg gctttgtggc ggcctagtga cagtacggta     5640
   tatcttccac caccttctgt ggccagagtt gtcagcactg atgattatgt gtctcgcaca     5700
   agcatatttt atcatgcagg cagttcccga ttattaactg taggcaatcc atattttagg     5760
   gttgtaccta atggtgcagg taataaacag gctgttccta aggtatccgc atatcagtat     5820
   agggtgttta gagtagcttt acccgatcct aataaatttg gattacctga ttctactata     5880
   tataatcctg aaacacaacg tttggtttgg gcatgtgtag gtatggaaat tggtcgtggg     5940
   cagcctttag gtattggcct aagtggccat ccattttata ataaattgga tgatacagaa     6000
   agtgctcatg cagctacagc tgttattacg caggatgtta gggataatgt gtcagttgat     6060
   tataagcaaa cacagctgtg tattttaggt tgtgtacctg ctattggtga gcactgggcc     6120
   aagggcacac tttgtaaacc tgcacaattg caacctggtg actgtcctcc tttggaactt     6180
   aaaaacacca ttattgagga tggtgatatg gtggatacag gttatggggc aatggatttt     6240
   agtacattgc aggatacaaa gtgcgaggtt ccattagaca tttgtcaatc catctgtaaa     6300
   tatccagatt atttgcaaat gtctgctgat ccctatgggg attctatgtt tttttgccta     6360
   cgccgtgaac aactgtttgc aagacatttt tggaataggg caggtgttat gggtgacaca     6420
   gtacctacgg acctatatat taaaggcact agcgctaata tgcgtgaaac ccctggcagt     6480
   tgtgtgtatt ccccttctcc cagtggctct attattactt ctgattctca attatttaat     6540
   aagccatatt ggttacataa ggcccagggc cataacaatg gtatttgttg gcataatcag     6600
   ttgtttgtta ctgtagtgga cactacccgc agtactaatt taacattatg tgcctctaca     6660
   caaaatcctg tgccaagtac atatgaccct actaagttta agcagtatag tagacatgtg     6720
   gaggaatatg atttacagtt tatttttcag ttgtgcacta ttactttaac tgcagaggtt     6780
   atgtcatata tccatagtat gaatagtagt atattagaaa attggaattt tggtgtccct     6840
   ccaccaccta ctacaagttt ggtggataca tatcgttttg tgcaatcagt tgctgttacc     6900
   tgtcaaaagg atactacacc tccagaaaag caggatccat atgataaatt aaagttttgg     6960
   actgttgacc taaaggaaaa attttcctcc gatttggatc aatatcccct tggtcgaaag     7020
   tttttagttc aggctgggtt acgtcgtagg cctaccatag gacctcgtaa gcgtcctgct     7080
   gcttccacgt ctactgcatc tactgcatct aggcctgcca aacgtgtacg tatacgtagt     7140
   aagaaataat atgttagcac atatatgtat gtttgtatgt atggttttgt atgttgtatg 7200
   tatgtatgta tttgtgtgat atattactgt attttgtttg tttgcgtgcg tgtatgtatg 7260
   aatgtgcctt gtggcatgta tggtgttact gtacataatt gtggtattaa ataaagtatg 7320
   ctaatagtgt tgtgtagggt tgcacccttg tgagtaacaa tactatttgt gtgtatgtgt 7380
   attgctttgt accctatatt ctttcctgta tttcaagtta taaacttgca tactacacag 7440
   catccatttt acttataatc ctccattttg ctgtgcaacc gatttcggtt gcctgtggct 7500
   tatatgtgac cttttaaaca taatacctaa actggcacat ttacaacccc tacatagttt 7560
   aacctactgg cgcgccttct tggcgtacat gtggcacacc tggtattagt cattttcctg 7620
   tccaggtgta ctaaaacaat ggcttgcaca actgtatcca caccctatgt aataaaactg 7680
   cttttaggca catattttag tctgttttta cctgtgctaa ttgtataatt ggcgtgtaga 7740
   accactttct tatccaacaa tctgtctact tgttacataa actataaact gactcactta 7800
   tacatacata gtttatgcaa ccgaaaaagg ttgggcccta taacacatac cttttctt 7858
<210> 16
   <211> 20
   <212> DNA
   <213> Artificial Sequence
<220>
   <223> pcr primer
<400> 16
   gaagagccaa ggacaggtac 20
<210> 17
   <211> 20
   <212> DNA
   <213> Artificial Sequence
<220>
   <223> pcr primer
<400> 17
   caacttcatc cacgttcacc 20
<210> 18
   <211> 23
   <212> DNA
   <213> Artificial Sequence
<220>
   <223> pcr probe
<400> 18
   ccctagggtt ggccaatcta ctc 23
<210> 19
   <211> 20
   <212> DNA
   <213> Artificial Sequence
<220>
   <223> pcr primer
<400> 19
   acacaactgt gttcactagc 20
<210> 20
   <211> 20
   <212> DNA
   <213> Artificial Sequence
<220>
   <223> pcr primer
<400> 20
   caacttcatc cacgttcacc 20
<210> 21
   <211> 30
   <212> DNA
   <213> Artificial Sequence
<220>
   <223> pcr probe
<400> 21
   tcaaacagac accatggtgc atctgactcc 30